# EUROPEAN PATENT APPLICATION

(11) **EP 1 908 462 A2**
(43) Date of publication of application: **09.04.2008**
(21) Application number: 07020297.3
(22) Date of filing: 20.06.2003
(51) Int. Cl.: A61K 31/137, A61K 31/138, A61K 31/554, A61K 31/704, A61K 38/16, A61K 38/49, A61K 45/06, A61P 9/04, A61P 9/10

(54) **Dichloroacetate in combination with cardioprotective or hemodynamic drugs**

(30) Priority: 20.06.2002 US 390709 P
(62) Divisional of application: 03739889.8
(71) Applicant: The Governors of the University of Alberta, Edmonton, Alberta T6G 2E1 (CA)
(72) Inventor: Lopaschuk, Gary D., Edmonton, Alberta T6G 2S2 (CA); Collins-Nakai, Ruth L., Edmonton, Alberta T6H 5R3 (CA)
(74) Representative: Vossius & Partner

(57) **Abstract**

The present invention provides compositions and methods for using cardioprotective or hemodynamic drugs in combination with dichloroacetate enabling usage of cardioprotective or hemodynamic drugs at concentrations higher than used in normal clinical practice without increasing deleterious side effects normally associated with the cardioprotective or hemodynamic drug, thereby conferring added clinical benefit. The present invention teaches administration of DCA with cardioprotective or hemodynamic drugs as an adjunct therapy thereby conferring added clinical benefit to clinically recommended protocols.

## Description

### FIELD OF INVENTION

The present invention relates to the field of cardiovascular disease and more particularly, the treatment of cardiac dysfunction with cardioprotective or hemodynamic drugs.

### BACKGROUND OF THE INVENTION

The heart is capable of utilizing a variety of energy substrates in order to meet its extremely high energy demands. The main fuels involved in maintaining cardiac function are glucose, lactate, and fatty acids. Under normal physiological conditions, a balance between fatty acid and carbohydrate utilization occurs, depending largely on the supply of either substrate. In situations where plasma fatty acid levels are elevated, such as diabetes mellitus or during a myocardial infarction, myocardial glucose oxidation decreases dramatically, and fatty acids become the dominant oxidative substrate. Experimental and clinical data have shown that increased fatty acid oxidation results in increased ischemic injury. Therapies for ischemic heart disease, which modulate myocardial metabolism, have been developed. One such metabolic modulator is dichloroacetate (DCA).

DCA is the prototype of a class of compounds known as "direct pyruvate dehydrogenase complex activators", or PDH activators. PDH converts pyruvate into acetyl CoA where it then enters the TCA cycle in the mitochondria. PDH kinase phosphorylates and inactivates PDH. DCA stimulates the PDH complex, by inhibiting the negative regulatory effects of PDH kinase. As a result, glucose and lactate oxidation increase and carbohydrate-derived mitochondrial acetyl CoA rises. Glucose oxidation is therefore increased at the expense of fatty acids (Lopaschuk G.D., et al. J Pharmacol Exp Ther. 264: 135-144 (1993); Lopaschuk, G.D., et al. Biochim Biophys Acta. 1213:263-276 (1994); Allard M.F., et al. Am J PhysioL 267:H742-H750 (1994)) via inhibition of both β-oxidation and of CPT-1 mediated fatty acyl-translocation. Following its description as a regulator of PDH activity in the isolated perfused heart, DCA was subsequently shown to reduce myocardial oxygen consumption in closed chest dogs and reduce indicators of ischemic stress during brief open-chest coronary occlusions in the same model (Mjos O.D et al. Cardiovasc Res 10:427-436 (1976)). In adult studies, it has been demonstrated that DCA administration significantly stimulates PDC in heart muscle, strongly suggesting that glucose oxidation is increased (Thannkikkotu B., et al (CABG). Can. J. CardioL 10: 130C(1994)). In a pilot project in which DCA was administered to pediatric patients, a significant drop in the requirements for inotropes in an immediate post operative period has been observed (Collins-Nakai R. et al Can. J. Cardiol. 11:106E(1995)).

Many pharmacological agents are currently being used to treat a wide variety of cardiovascular disorders. Some of the classes of compounds that have had the most success are digitalis glycosides, inotropes, beta-blockers and calcium channel blockers. An example of a cardiac glycoside is digoxin. Digoxin causes a reversible inhibition of myocardial membrane bound (Na⁺ + K⁺)-ATPase. Intracellular accumulation of Na⁺ (and decrease in intracellular K⁺) promotes Ca²⁺ entry into the cell. Subsequent release of Ca²⁺ from sarcoplasmic reticulum causes a positive inotrope effect, the primary therapeutic action. A class of compounds with similar actions (i.e. which alter Ca²⁺ influx) are calcium channel blockers (calcium entry blocker or calcium antagonists). When an effective stimulus is applied to a muscle cell the result is an influx of Ca²⁺, which in turn triggers the intracellular events leading to muscle contraction.

Several different types of antagonists, such as diltiazem can block this sequence of Ca²⁺ dependent steps. In the case of unstable angina for example, diltiazem has been shown to be an effective treatment, possibly due to the suppression of coronary vasospasm. Another class of compounds which alter Ca²⁺ influx is β₁-adrenoreceptor agonists (catecholamine). When β-receptors are stimulated by a catecholamine, such as dobutamine, they react with a stimulatory guanine-nucleotide-binding regulatory protein (Gₛ) present in the cell membrane. Gₛ binds with guanosine triphosphate (GTP) to from a complex that stimulates adenylate cyclase activity and catalyzes the formation of intracellular cyclic AMP. Cyclic AMP combines with a protein kinase that catalyzes the phosphorylation of specific enzymes, the end result of which are elevated Ca²⁺ levels in cardiac and other cells. Dobutamine has a positive inotropic effect on the heart through stimulation of β-receptors. In clinical studies, the action of dobutamine on the heart is unique in that it increases the force of contraction without increasing the heart rate significantly.

A selective antagonist of β₁-adrenoreceptors is metoprolol. β₁-adrenoreceptor antagonists are used extensively in the treatment of cardiovascular diseases, e.g., hypertension, angina pectoris, cardiac arrhythmias and in the secondary prevention of myocardial infarction and sudden death in patients with coronary thrombosis. Metoprolol has been used in the prophylaxis of angina pectoris and in the treatment of hypertension. Furthermore there has been demonstrations of the ability of metabolic agents to function in the presence of these beneficial pharmacological agents (Cross, H.R Exp. Opin. Pharmacother. 2:857-875 (2001)).

Current therapies aimed at improving contractile function often involve the use of inotropes such as calcium, dopamine, epinephrine, ephedrine, phenylephrine, and dobutamine. Although agents such as dobutamine have been demonstrated to increase cardiac work, they have also been demonstrated to increase cardiac oxygen consumption and therefore may not enhance overall mechanical efficiency (Bersin, R.M., et al. JACC 23(7):1617-1624 (1994)), particularly since those patients requiring the drugs are generally in situations of reduced blood flow or circulation leading to reduced availability of oxygen to the cardiac environment. The potential for inotropes or drugs with inotropic effect to increase oxygen consumption to a greater extent than contractile function has been termed an oxygen wasting effect (Chandler, B.Met al. Circ. Res. 22:729-735 (1968); Suga Het al. Circ Res. 53:306-318 (1983)). Inotropic drugs are also reportedly associated with increases in intracellular calcium concentration and heart rate, which may also be potentially harmful, especially in hearts with impaired energy balance (Hasenfuss, G et al. 94:3155-3160 (1996)).

### SUMMARY OF THE INVENTION

The present invention provides for a method to ameliorate the negative side effects of a serum concentration of a cardioprotective or hemodynamic drug higher than that used in normal clinical practice; through administration of DCA prior to, simultaneous with or subsequent to administration of the cardioprotective or hemodynamic drug.

According to a further aspect of the present invention, the amelioration of negative side effects results from the metabolic effects of DCA administered prior to, simultaneous with or subsequent to administration of the cardioprotective or hemodynamic drug.

A further aspect of the present invention provides for the metabolic effects of DCA administered prior to, simultaneous with or subsequent to administration of the cardioprotective or hemodynamic drug to include an increase in glucose metabolism.

The present invention provides for a composition of a unit dosage form of DCA and a cardioprotective or hemodynamic drug wherein the cardioprotective or hemodynamic drug attains a serum concentration greater than that which would be attained in normal clinical practice. According to this aspect of the invention, the composition may be used in patients in need of treatment without substantial increase in side effects compared to the use of cardioprotective or hemodynamic drug alone at concentrations used in normal clinical practice.

According to one aspect, the present invention provides for a composition of said unit dosage form of DCA with a cardioprotective or hemodynamic drug, where the cardioprotective or hemodynamic drug is a Na⁺/K⁺ ATPase inhibitor.

A further aspect of the present invention provides for a composition of said unit dosage form of DCA with a Na⁺/K⁺ ATPase inhibitor, where the Na⁺/K⁺ ATPase inhibitor is digoxin.

A further aspect of the present invention provides for a composition of said unit dosage form of DCA with digoxin, where the digoxin attains a serum concentration of greater than 2.5nM.

A further aspect of the present invention provides for a composition of said unit dosage form of DCA with digoxin, where the digoxin attains a serum concentration of between 2.5nM to 10.0nM.

According to one aspect, the present invention provides for a composition of said unit dosage form of DCA with a cardioprotective or hemodynamic drug, where the cardioprotective or hemodynamic drug is a calcium channel blocker.

A further aspect of the present invention provides for a composition of said unit dosage form of DCA with a calcium channel blocker, where the calcium channel blocker is diltiazem.

A further aspect of the present invention provides for a composition of said unit dosage form of DCA with diltiazem, where the diltiazem attains a serum concentration of greater than 0.5µM.

A further aspect of the present invention provides for a composition of said unit dosage form of DCA with diltiazem where, the diltiazem attains a serum concentration of between 0.5µM to 5.0µM.

According to one aspect, the present invention provides for a composition of said unit dosage form of DCA with a cardioprotective or hemodynamic drug where, the cardioprotective or hemodynamic drug is a β₁-adrenoreceptor agonist.

A further aspect of the present invention provides for a composition of said unit dosage form of DCA with a β₁-adrenoreceptor agonist, where the β₁-adrenoreceptor agonist is dobutamine.

A further aspect of the present invention provides for a composition of said unit dosage form of DCA with dobutamine, where the dobutamine attains a serum concentration of greater than 0.6µM.

A further aspect of the present invention provides for a composition of said unit dosage form of DCA with dobutamine, where the dobutamine attains a serum concentration of between 0.6µM to 5.0µM.

According to one aspect, the present invention provides for a composition of said unit dosage form of DCA with a cardioprotective or hemodynamic drug, where the cardioprotective or hemodynamic drug is a β₁- adrenoreceptor antagonist.

A further aspect of the present invention provides for a composition of said unit dosage form of DCA with a β₁- adrenoreceptor antagonist, where the β₁- adrenoreceptor antagonist is metoprolol.

A further aspect of the present invention provides for a composition of said unit dosage form of DCA with metoprolol, where the metoprolol attains a serum concentration of greater than 0.4µM.

A further aspect of the present invention provides for a composition of said unit dosage form of DCA with metoprolol, where the metoprolol attains a serum concentration of between 0.4µM to 5.0µM.

According to one aspect, the present invention provides for a composition of said unit dosage form of DCA with a cardioprotective or hemodynamic drug, where the cardioprotective or hemodynamic drug is a thrombolytic agent.

A further aspect of the present invention provides for a composition of said unit dosage form of DCA with a thrombolytic agent, where the thrombolytic agent is tissue plasminogen activator or streptokinase.

An aspect of the present invention provides for a composition comprising said unit dosage form of DCA and a cardioprotective or hemodynamic drug where the cardioprotective or hemodynamic drug decreases heart rate, decreases arrhythmia, decreases vasospasm, decreases fatty acid oxidation, increases contractile force or increases coronary blood flow.

An aspect of the present invention provides for a kit comprising said unit dosage form of DCA and a cardioprotective or hemodynamic drug, and instructions sufficient to enable one skilled in the art to administer the DCA and cardioprotective or hemodynamic drug to give a cardioprotective or hemodynamic effect.

A further aspect of the present invention provides for a kit comprising said unit dosage form of DCA and cardioprotective or hemodynamic drug, packaged individually.

A further aspect of the present invention provides for a kit comprising said unit dosage form of DCA and cardioprotective or hemodynamic drug.

An aspect of the present invention provides for a kit comprising said unit dosage form of DCA and a cardioprotective or hemodynamic drug, where the cardioprotective or hemodynamic drug is a Na⁺/K⁺ ATPase inhibitor.

A further aspect of the present invention provides for a kit comprising said unit dosage form of DCA and a Na⁺/K⁺ ATPase inhibitor, where the Na⁺/K⁺ ATPase inhibitor is digoxin.

An aspect of the present invention provides for a kit comprising said unit dosage form of DCA and a cardioprotective or hemodynamic drug, where the cardioprotective or hemodynamic drug is a calcium channel blocker.

A further aspect of the present invention provides for a kit comprising said unit dosage form of DCA and a calcium channel blocker; where the calcium channel blocker is diltiazem.

An aspect of the present invention provides for a kit comprising said unit dosage form of DCA and a cardioprotective or hemodynamic drug, where the cardioprotective or hemodynamic drug is a β₁-adrenoreceptor agonist.

A further aspect of the present invention provides for a kit comprising said unit dosage form of DCA and a β₁-adrenoreceptor agonist, where the β₁-adrenoreceptor agonist is dobutamine.

An aspect of the present invention provides for a kit comprising said unit dosage form of DCA and a cardioprotective or hemodynamic drug, where the cardioprotective or hemodynamic drug is a β₁-adrenoreceptor antagonist

A further aspect of the present invention provides for a kit comprising said unit dosage form of DCA and a β₁-adrenoreceptor antagonist, where the β₁-adrenoreceptor antagonist is metoprolol.

According to one aspect, the present invention provides for kit comprising said unit dosage form of DCA and a cardioprotective or hemodynamic drug, where the cardioprotective or hemodynamic drug is a thrombolytic agent.

A further aspect of the present invention provides for a kit comprising said unit dosage form of DCA and a thrombolytic agent, where the thrombolytic agent is tissue plasminogen activator or streptokinase.

An aspect of the present invention provides for a method of treating a subject in need of cardioprotective of hemodynamic drugs, where the cardioprotective or hemodynamic drug can attain a higher serum concentration than that used in normal clinical practice.

According to one aspect, the present invention provides for a method of treating a subject in which DCA is co-administered with a cardioprotective or hemodynamic drug which attains a serum concentration greater than that used in normal clinical practice, where the cardioprotective or hemodynamic drug is a Na⁺/K⁺ ATPase inhibitor.

A further aspect of the present invention provides for a method of treating a subject in which DCA is co-administered with a Na⁺/K⁺ ATPase inhibitor which attains a serum concentration greater than that used in normal clinical practice, where the Na⁺/K⁺ ATPase inhibitor is digoxin.

A further aspect of the present invention provides for a method of treating a subject in which DCA is co-administered with digoxin, where the digoxin attains a serum concentration of greater than 2.5nM.

A further aspect of the present invention provides for a method of treating a subject in which DCA is co-administered with digoxin, where the digoxin attains a serum concentration ofbetween 2.5nM to 10.0nM.

According to one aspect, the present invention provides for a method of treating a subject in which DCA is co-administered with a cardioprotective or hemodynamic drug which attains a serum concentration greater than that used in normal clinical practice, where the cardioprotective or hemodynamic drug is a calcium channel blocker.

A further aspect of the present invention provides for a method of treating a subject in which DCA is co-administered with a calcium channel blocker which attains a serum concentration greater than that used in normal clinical practice, where the calcium channel blocker is diltiazem.

A further aspect of the present invention provides for a method of treating a subject in which DCA is co-administered with diltiazem, where the diltiazem attains a serum concentration of greater than 0.5µM.

A further aspect of the present invention provides for a method of treating a subject in which DCA is co-administered with diltiazem, where the diltiazem attains a serum concentration of between 0.5µM to 5.0µM.

According to one aspect, the present invention provides for a method of treating a subject in which DCA is co-administered with a cardioprotective or hemodynamic drug which attains a serum concentration greater than that used in normal clinical practice, where the cardioprotective or hemodynamic drug is a β₁-adrenoreceptor agonist.

A further aspect of the present invention provides for a method of treating a subject in which DCA is co-administered with a β₁-adrenoreceptor agonist which attains a serum concentration greater than that used in normal clinical practice, where the β₁-adrenoreceptor agonist is dobutamine.

A further aspect of the present invention provides for a method of treating a subject in which DCA is co-administered with dobutamine, where the dobutamine attains a serum concentration of greater than 0.6µM.

A further aspect of the present invention provides for a method of treating a subject in which DCA is co-administered with dobutamine, where the dobutamine attains a serum concentration of between 0.6µM to 5.0µM.

According to one aspect, the present invention provides for a method of treating a subject in which DCA is co-administered with a cardioprotective or hemodynamic drug which attains a serum concentration greater than that used in normal clinical practice, where the cardioprotective or hemodynamic drug is a β₁-adrenoreceptor antagonist.

A further aspect of the present invention provides for a method of treating a subject in which DCA is co-administered with a β₁-adrenoreceptor antagonist which attains a serum concentration greater than that used in normal clinical practice, where the β₁-adrenoreceptor antagonist is metoprolol.

A further aspect of the present invention provides for a method of treating a subj ect in which DCA is co-administered with metoprolol, where the metoprolol attains a serum concentration of greater than 0.4µM.

A further aspect of the present invention provides for a method of treating a subject in which DCA is co-administered with metoprolol, where the metoprolol attains a serum concentration of between 0.4µM to 5.0µM.

According to one aspect, the present invention provides for a method of treating a subject in which DCA is co-administered with a cardioprotective or hemodynamic drug which attains a serum concentration greater than that used in normal clinical practice, where the cardioprotective or hemodynamic drug is a thrombolytic agent.

A further aspect of the present invention provides for a method of treating a subject in which DCA is co-administered with a β₁-adrenoreceptor antagonist which attains a serum concentration greater than that used in normal clinical practice, where the thrombolytic agent is tissue plasminogen activator or streptokinase.

An aspect of the present invention provides for a method of treating a subject in which DCA is co-administered with cardioprotective or hemodynamic drugs which attain a serum concentration greater than that used in normal clinical practice, where the cardioprotective or hemodynamic drug decreases heart rate, decreases arrhythmia, decreases vasospasm, decreases fatty acid oxidation, increases contractile force or increases coronary blood flow.

An aspect of the present invention provides for a method of treating a subject in which DCA is co-administered with cardioprotective or hemodynamic drugs which attain a serum concentration greater than that used in normal clinical practice, where the subject is at risk for hypertension, coronary ischemia, angina pectoris, arrhythmia, coronary thrombosis, myocardial infarction or enlarged heart.

An aspect of the present invention provides for a method of treating a subject in which DCA is co-administered with cardioprotective or hemodynamic drugs which attain a serum concentration greater than that used in normal clinical practice, where the subject is experiencing an ischemic event.

An aspect of the present invention provides for a method of treating a subject in which DCA is co-administered with cardioprotective or hemodynamic drugs which attain a serum concentration greater than that used in normal clinical practice, where the subject is recovering from an ischemic event.

A further aspect of the present invention provides for the ischemic event to result from a surgical procedure.

A further aspect of the present invention provides for the surgical procedure to be heart surgery.

An aspect of the present invention provides for methods of increasing the efficacy of a cardioprotective or hemodynamic drug without substantially increasing the side effects, by administration of cardioprotective or hemodynamic drugs which attain a serum concentration in the patient greater than that which would be attained in normal clinical practice, wherein DCA is administered prior to, simultaneous with or subsequent to the cardioprotective or hemodynamic drug.

An aspect of the present invention provides for methods of increasing the efficacy of a cardioprotective or hemodynamic drug without substantially increasing the side effects, by administration of cardioprotective or hemodynamic drugs which attain a serum concentration in the patient greater than that which would be attained in normal clinical practice, wherein DCA is administered as a combination or adjuvant therapy.

An aspect of the present invention provides for methods of increasing the ability to use higher than clinical levels of cardioprotective or hemodynamic drugs without substantially increasing the side effects, by administration of cardioprotective or hemodynamic drugs which attain a serum concentration in the patient greater than that which could be attained in normal clinical practice, wherein DCA is administered prior to, simultaneous with or subsequent to the cardioprotective or hemodynamic drug.

An aspect of the present invention provides for methods of increasing the ability to use higher than clinical levels of cardioprotective or hemodynamic drugs without substantially increasing the side effects, by administration of cardioprotective or hemodynamic drugs which attain a serum concentration in the patient greater than that which could be attained in normal clinical practice, wherein DCA is administered as a combination or adjuvant therapy.

According to an aspect, the present invention provides for combination therapy of DCA with cardioprotective or hemodynamic drugs, enabling administration of higher amounts of cardioprotective or hemodynamic drugs than used in normal clinical practice.

One aspect of the present invention is directed to a method of increasing the amount of cardioprotective or hemodynamic drugs capable of being administered to a patient without increasing the negative side effects associated with the cardioprotective or hemodynamic drug, which comprises administration to said patient amounts of cardioprotective or hemodynamic drugs, at greater than that used in normal clinical practice, in combination with an amount of DCA sufficient to diminish or attenuate the negative side effects arising from the higher amount of cardioprotective or hemodynamic drug.

A further aspect of the present invention provides for a method of administration of DCA in combination with amounts of cardioprotective or hemodynamic drug at greater than that used in normal clinical practice, where the DCA achieves a concentration of 1mM to 3mM in the patient.

A further aspect of the present invention provides for a method of administration of DCA in combination with amounts of cardioprotective or hemodynamic drug at greater than that used in normal clinical practice, where the DCA achieves a concentration of 2mM in the patient.

An aspect of the present invention provides for a method of treating a subject in which DCA is administered prior to, simultaneous with or subsequent to a cardioprotective or hemodynamic drug.

A further aspect of the present invention provides for a method of treating a subject in which DCA is administered prior to, simultaneous with or subsequent to a cardioprotective or hemodynamic drug, where the cardioprotective or hemodynamic drug is a Na+/K+ ATPase.

A further aspect of the present invention provides for a method of treating a subject in which DCA is administered prior to, simultaneous with or subsequent to a Na+/K+ ATPase, where the Na+/K+ ATPase is digoxin.

A further aspect of the present invention provides for a method of treating a subject in which DCA is administered prior to, simultaneous with or subsequent to a Na+/K+ ATPase; in which the subject would benefit from an increase in cardiac efficiency.

A further aspect of the present invention provides for a method of treating a subject in which DCA is administered prior to, simultaneous with or subsequent to a Na+/K+ ATPase; in which the subject would benefit from a decrease in oxygen consumption.

While the present invention is not limited to a particular dose level of DCA, doses and dosing protocols suitable for the methods of the present invention would be arrived at without experimentation, particularly with reference paid to PCT International Application PCT/US98/20394 "Postsurgical Treatment with Dichloroacetate" or United States Utility Patent Application, 10/268,069 "Methods of Cardioprotection Using Dichloroacetate in Combination with an Inotrope", both of which are herein incorporated by reference.

### DEFINITIONS

The following definitions are to be used to further explain the invention and should in no way be used to limit the scope of the present invention.

"Unit dosage form" as used herein refers to physically discrete units, suitable as unit dosages, each unit containing a predetermined quantity of active ingredient calculated to produce the desired therapeutic effect in association with the required pharmaceutical carrier.

"Normal clinical practice" as used herein refers to that currently known in the art, or determinable from the art, as proper and reasonable practice and care in a clinical setting giving due consideration and attention to the individual patient's care, safety and clinical outcome.

"Serum concentration" as used herein refers to the amount of drug of interest in the serum of a subject, wherein the amount of drug is determined in accordance with methods known to the art or determinable from the art.

"Thrombolytic" as used herein refers to a member of the class of drug referred to in the art as thrombolytic agents or drugs having the ability to directly or indirectly dissolve, reduce or remove cardiac or cardiac related blockages arising from blood clots or other naturally occurring blockages.

### BRIEF DESCRIPTION OF THE DRAWINGS

Figure 1 is a graph of the rate of glucose oxidation in isolated rat hearts with administration of 2mM DCA, 3nM digoxin, 3nM digoxin with 2mM DCA, and a control (0.05% DMSO).
Figure 2 is a graph of the rate of glycolysis in isolated rat hearts with administration of 2mM DCA, 3nM digoxin, 3nM digoxin with 2mM DCA, and a control (0.05% DMSO).
Figure 3 is a graph of the rate of glucose oxidation in isolated rat hearts with administration of 2mM DCA, 0.8 µM diltiazem, 0.8 µM diltiazem with 2mM DCA, and a control (0.05% DMSO).
Figure 4 is a graph of the rate of glycolysis in isolated rat hearts with administration of 2mM DCA, 0.8 µM diltiazem, 0.8 µM diltiazem with 2mM DCA, and a control (0.05% DMSO).
Figure 5 is a graph of the rate of glucose oxidation in isolated rat hearts with administration of 2mM DCA, 1 µM dobutamine, 1 µM dobutamine with 2mM DCA, and a control (0.05% DMSO).
Figure 6 is a graph of the rate of glycolysis in isolated rat hearts with administration of 2mM DCA, 1 µM dobutamine, 1 µM dobutamine with 2mM DCA, and a control (0.05% DMSO).
Figure 7 is a graph of the rate of glucose oxidation of isolated rat hearts with administration of 2mM DCA, 1 µM metoprolol, 1 µM metoprolol with 2mM DCA, and a control (0.05% DMSO).
Figure 8 is a graph of the rate of glycolysis in isolated rat hearts with administration of 2mM DCA, 1 µM metoprolol, 1 µM metoprolol with 2mM DCA, and a control (0.05% DMSO).
Figure 9 is a graph of the rate of glucose oxidation in isolated rat hearts subjected to global ischemia with administration of 2mM DCA, 3nM digoxin, 3nM digoxin with 2mM DCA, and control (0.05% DMSO).
Figure 10 is a graph of the rate of glycolysis in isolated rat hearts subjected to global ischemia with administration of 2mM DCA, 3nM digoxin, 3nM digoxin with 2mM DCA, and control (0.05% DMSO).
Figure 11 is a graph of cardiac function in isolated rat hearts subjected to global ischemia with administration of 2mM DCA, 3nM digoxin, 3nM digoxin with 2mM DCA, and control (0.05% DMSO).
Figure 12 is a graph of cardiac work in isolated rat hearts subjected to global ischemia with administration of 2mM DCA, 3nM digoxiri, 3nM digoxin with 2mM DCA, and control (0.05% DMSO).
Figure 13 is a graph of oxygen consumption in isolated rat hearts subjected to global ischemia with administration of 2mM DCA, 3nM digoxin, 3nM digoxin with 2mM DCA, and control (0.05% DMSO).
Figure 14 is a graph of cardiac efficiency in isolated rat hearts subjected to global ischemia with administration of 2mM DCA, 3nM digoxin, 3nM digoxin with 2mM DCA, and control (0.05% DMSO).
Figure 15 is a graph of the rate of glucose oxidation in isolated rat hearts subjected to global ischemia with administration of 2mM DCA, 1 mM metoprolol, 1 mM metoprolol with 2mM DCA, and a control (0.05% DMSO).
Figure 16 is a graph of the rate of glycolysis in isolated rat hearts subjected to global ischemia with administration of 2mM DCA, 1 mM metoprolol, 1 mM metoprolol with 2mM DCA, and a control (0.05% DMSO).
Figure 17 is a graph of cardiac function in isolated rat hearts subjected to global ischemia with administration of 2mM DCA, 1 mM metoprolol, 1 mM metoprolol with 2mM DCA, and a control (0.05% DMSO).
Figure 18 is a graph of cardiac work in isolated rat hearts subjected to global ischemia with administration of 2mM DCA, 1 mM metoprolol, 1 mM metoprolol with 2mM DCA; and a control (0.05% DMSO).
Figure 19 is a graph of oxygen consumption in isolated rat hearts subjected to global ischemia with administration of 2mM DCA, 1 mM metoprolol, 1 mM metoprolol with 2mM DCA, and a control (0.05% DMSO).
Figure 20 is a graph of cardiac efficiency in isolated rat hearts subjected to global ischemia with administration of 2mM DCA, 1 mM metoprolol, 1 mM metoprolol with 2mM DCA, and a control (0.05% DMSO).
Figure 21 is a graph of the rate of glucose oxidation in isolated rat hearts subjected to low flow demand ischemia with administration of 2mM DCA, 0.8 mM diltiazem, 0.8 mM diltiazem with 2mM DCA, and a control (0.05% DMSO).
Figure 22 is a graph of the rate of glycolysis in isolated rat hearts subjected to low flow demand ischemia with administration of 2mM DCA, 0.8 mM diltiazem, 0.8 mM diltiazem with 2mM DCA, and a control (0.05% DMSO).
Figure 23 is a graph of cardiac function in isolated rat hearts subjected to low flow demand ischemia with administration of 2mM DCA, 0.8 mM diltiazem, 0.8 mM diltiazem with 2mM DCA, and a control (0.05% DMSO).
Figure 24 is a graph of cardiac work in isolated rat hearts subjected to low flow demand ischemia with administration of 2mM DCA, 0.8 mM diltiazem, 0.8 mM diltiazem with 2mM DCA, and a control (0.05% DMSO).
Figure 25 is a graph of oxygen consumption in isolated rat hearts subjected to low flow demand ischemia with administration of 2mM DCA, 0.8 mM diltiazem, 0.8 mM diltiazem with 2mM DCA, and a control (0.05% DMSO).
Figure 26 is a graph of cardiac efficiency in isolated rat hearts subjected to low flow demand ischemia with administration of 2mM DCA, 0.8 mM diltiazem, 0.8 mM diltiazem with 2mM DCA, and a control (0.05% DMSO).
Figure 27 is a graph of the rate of glucose oxidation in isolated rat hearts subjected to low flow demand ischemia with administration of 2mM DCA, 1 mM metoprolol, 1 mM metoprolol with 2mM DCA, and a control (0.05% DMSO).
Figure 28 is a graph of the rate of glycolysis in isolated rat hearts subjected to low flow demand ischemia with administration of 2mM DCA, 1 mM metoprolol, 1 mM metoprolol with 2mM DCA, and a control (0.05% DMSO).
Figure 29 is a graph of cardiac function in isolated rat hearts subjected to low flow demand ischemia with administration of 2mM DCA, 1 mM metoprolol, 1 mM metoprolol with 2mM DCA, and a control (0.05% DMSO).
Figure 30 is a graph of cardiac work in isolated rat hearts subjected to low flow demand ischemia with administration of 2mM DCA, 1 mM metoprolol, 1 mM metoprolol with 2mM DCA, and a control (0.05% DMSO).
Figure 31 is a graph of oxygen consumption in isolated rat hearts subjected to low flow demand ischemia with administration of 2mM DCA, 1 mM metoprolol, 1 mM metoprolol with 2mM DCA, and a control (0.05% DMSO).
Figure 32 is a graph of cardiac efficiency in isolated rat hearts subjected to low flow demand ischemia with administration of 2mM DCA, 1 mM metoprolol, 1 mM metoprolol with 2mM DCA, and a control (0.05% DMSO).

### DETAILED DESCRIPTION OF THE INVENTION

Repetitive contraction of cardiac muscle requires an efficient and ready source of ATP production to sustain mechanical activity. There are two main mechanisms to produce this ATP in cardiac muscle: 1) glycolysis utilizing glucose as a substrate; and 2) oxidative metabolism utilizing lactate, glucose or fatty acids as substrates. Glycolysis is an anaerobic process and produces 2ATP per mole of glucose converted to pyruvate. Fatty acid, lactate and glucose oxidation are aerobic processes, that is, requiring oxygen, and produce 129 moles of ATP, 18 moles of ATP and 36 moles of ATP per mole of substrate metabolized, respectively. Bing *et al* identified that the adult human heart primarily utilizes glucose, lactate and fatty acids as the major sources of energy (Bing, R.J., et al. Am. J. Med. 15:284-296 (1953)). The type of energy substrate used by the heart can have a profound impact on the ability of the heart to withstand an episode of hypoxia or ischemia (Lopaschuk, G.D., et al. Circ. Res. 63(6): 1036-1043 (1988)). As a result, changes in energy substrate preference during maturation of the heart should influence the outcome of hypoxia or ischemia.

Under non-ischemic conditions, as noted previously, fatty acids are the primary energy substrate in the adult heart, with glucose oxidation providing only 30 to 40 percent of myocardial ATP production. In experimental studies, it has been demonstrated that glucose oxidation provides an even smaller portion of ATP production in hearts reperfused following a period of global ischemia (Lopaschuk, G.D et al. Circ. Res. 66:546-553 (1990)). One of the primary factors resulting in low glucose oxidation rates post-ischemia is the circulating level of fatty acids: serum fatty acids are potent inhibitors of myocardial glucose oxidation.

In.patients suffering a myocardial infarction or undergoing heart surgery, serum fatty acids can be markedly elevated (Lopaschuk, G.D., et al. Am. Heart J., 128:61-67 (1994)). These high levels of fatty acids have been shown to potentiate ischemic injury in several experimental models including pig, dog, rabbit and rat hearts (Saddik, M., et al. J. Biol. Chem. 266:8162-8170 (1991)). In both aerobic and reperfused ischemic rat hearts, high levels of fatty acids markedly inhibit glucose oxidation rates. This is believed to be the result of marked inhibition by fatty acids of the pyruvate dehydrogenase complex (PDC), a key enzyme complex regulating carbohydrate oxidation.

It is further believed that overcoming fatty acid inhibition of PDC will dramatically increase glucose oxidation and improve functional recovery of ischemic hearts. One of the pharmacological agents that is particularly effective in reversing fatty acid inhibition of PDC is dichloroacetate. Dichloroacetate (DCA) directly stimulates PDC, resulting in a marked stimulation of glucose oxidation (McVeigh, J.J et al. (1990) Am. J. Physiol. 259:H1079-1085).

In this investigation we studied the metabolic effects of DCA in combination with a Na⁺/K⁺ ATPase inhibitor (digoxin), a β₁-adrenoreceptor agonist (dobutamine), a β₁-adrenoreceptor antagonist (metoprolol) and a calcium channel blocker (diltiazem) in the perfused rat heart. We investigated whether the stimulation of glucose metabolism by DCA could be maintained in the presence of these agents. By being able to stimulate glucose oxidation using DCA in conjunction with the combined effect of the previously mentioned compounds, it is hoped that a new type of therapy for the treatment of heart disease may be found.

### EXAMPLES

### METHODS USED

The studies described in Examples 1-9 utilized the methods described as follows. Dosage of each cardioprotective or hemodynamic drug used is based on the equivalent administration of the maximum dosage allowed for the maximum physiological effect in clinically recommended protocols for the treatment of human patients.

### Isolated Rat Heart Model

Rat hearts were cannulated for isolated working heart perfusions as described in "An imbalance between glycolysis and glucose oxidation is a possible explanation for the detrimental effects of high levels of fatty acids during aerobic reperfusion of ischemic hearts.", (J Pharmacol Exp Ther. 264:135 (1993); herein incorporated by reference. In brief, male Sprague-Dawley rats (0.3-0.35 kg) were anesthetized with pentobarbital sodium (60 mg/kg IP) and hearts were quickly excised, the aorta was cannulated and a retrograde perfusion at 37°C was initiated at a hydrostatic pressure of 60 mm Hg. Hearts were trimmed of excess tissue, and the pulmonary artery and the opening to the left atrium were then cannulated. After 15 min of Langendorff perfusion, hearts were switched to the working mode by clamping the aortic inflow line from the Langendorff reservoir and opening the left atrial inflow line. The perfusate was delivered from an oxygenator into the left atrium at a constant preload pressure of 11 mm Hg. Perfusate was ejected from spontaneously beating hearts into a compliance chamber (containing 1 ml of air) and into the aortic outflow line. The afterload was set at a hydrostatic pressure of 80 mm Hg. All working hearts were perfused with Krebs'-Henseleit solution containing calcium 2.5 mmol/L, glucose 5.5 mmol/L, 3% bovine serum albumin (Fraction V, Boehringer Mannheim), and with 1.2 mmol/L palmitate. Palmitate was bound to the albumin as described previously (Saddik M., et al. J Biol. Chem. 267:3825-3831 (1992)). The perfusate was recirculated, and pH was adjusted to 7.4 by bubbling with a mixture containing 95% O₂ and 5% CO₂. For the aerobic model hearts, all tested compounds were introduced into the heart 5 minutes into the working mode following Langendorff Perfusion.

Spontaneously beating hearts were used in all perfusions. Heart rate and aortic pressure were measured with a Biopac Systems Inc. blood pressure transducer connected to the aortic outflow line. Cardiac output and aortic flow were measured with Transonic T206 ultrasonic flow probes in the preload and afterload lines, respectively. Coronary flow was calculated as the difference between cardiac output and aortic flow. The O₂ contents of the perfusate entering and leaving the heart were measured using YSI^{™} micro oxygen electrodes placed in the preload and pulmonary arterial lines, respectively. Myocardial O₂ consumption (MVO₂) was calculated according to the Fick principle, using coronary flow rates and the arteriovenous difference in perfusate O₂ concentration. Cardiac work was calculated as the product of systolic pressure and cardiac output. Cardiac efficiency was defined as a ratio of cardiac work to MVO₂.

Hearts that were subjected to global ischemia were initially aerobically perfused for 30 minutes, and then subjected to 30 minutes of global no flow ischemia by clamping the left atrial inflow line and the aortic outflow line. This was followed by 60 minutes of reperfusion, which was produced by removing the clamps from the left atrial inflow line and the aortic outflow line. All tested compounds were introduced into the perfusate five minutes prior to reperfusion.

Hearts that were subjected to a low flow demand ischemia were first perfused aerobically for 30 minutes then a low flow demand ischemia was induced by attenuating coronary flow with a diastolic backflow controller located in the aortic outflow line. Using our low flow working rat heart model we are able to achieve a drop of approximately 50% in coronary flow while still subjecting the hearts to the same afterload (i.e. maintaining cardiac work). All tested compounds were introduced into the perfusate five minutes into initiation of aerobic perfusion.

### Measurement of Glycolysis and Glucose Oxidation.

Glycolysis and glucose oxidation were measured simultaneously by perfusing hearts with [5-³H/U-¹⁴C] glucose (Liu H., et al. Am J PhysioL 270:H72-H80 (1996); Taegtmeyer Het al. Am J CardioL 80:3A-10A (1997)). The total myocardial ³H₂O production and ¹⁴CO₂ production were determined at 10 minute intervals for the entirety of the aerobic period, 60 minutes for normal hearts and 30 minutes for the global ischemia and low flow demand ischemia models. In the global ischemia model, the total myocardial ³H₂O and ¹⁴CO₂ production were determined at 20 minute intervals for the 60 minute reperfusion period. In the low flow demand ischemia model, the total myocardial ³H₂O and ¹⁴CO₂ production were determined at 10 minute intervals for the 30 minute reperfusion period. Glucose oxidation rates were determined by quantitative measurement of ¹⁴CO₂ production as described previously (Barbour R.L., et al. Biochemistr. 1923:6503-6062 (1984)).

### EXAMPLE 1

### Effects of DCA (2 mM) on cardiac function and efficiency in normal hearts.

As can be seen in Table 1 treatment with DCA had no significant effect on heart rate, peak systolic pressure or heart rate x peak systolic pressure (HR x PSP). In Table 2, treatment with DCA shows that there was no effect on any functional parameters, nor were there any differences in O₂ consumption or cardiac efficiency.

**Table 1: Effect of various drugs on heart rate, peak systolic pressure and heart function**

| | **Condition** | **Heart Rate** | **Peak Systolic Pressure** | **HRxPSP** |
|---|---|---|---|---|
| | | **(beats•min⁻¹)** | **(mmHg)** | **(beats•min⁻¹• mmHg•10⁻²⁾** |
| **AEROBICALLY PERFUSED** | Control (0.05%DMSO) | 239 ± 12 | 137 ± 5 | 32 ± 1 |
| | DCA (2mM) | 239 ± 8 | 125 ± 3 | 32 ± 1 |
| | Diltiazem (0.8 µM) | 202 ± 3 | 127 ± 4 | 25 ± 1 |
| | +DCA (2mM) | 230 ± 19 | 131 ± 9 | 29 ± 3 |
| | Digoxin (3nM) | 258 ± 11 | 129 ± 4 | 32 ± 1 |
| | +DCA (2mM) | 254 ± 7 | 121 ± 1 | 30 ± 1 |
| | Metoprolol (1 µM) | 250 ± 26 | 130 ± 2 | 32 ± 4 |
| | +DCA(2mM) | 40 ± 6 | 129 ± 5 | 30 ± 1 |
| | Dobutamine (1 µM) | 333 ± 7 | 124 ± 3 | 40 ± 2 |
| | +DCA (2 mM) | 312 ± 14 | 130 ± 6 | 41 ± 4 |
| **GLOBAL ISCHEMIA** | Aerobic Control (0.05%DMSO) | 247 + 14 | 123 ± 4 | 30 ± |
| | Post-Ischemic Control (0.05%DMSO) | 149 ±21 | 58±11 | 86±3 |
| | DCA(2mM) | 208 ± 1 | 87±9 9 | 18±2 |
| | Digoxin (3 nM) | 207 ± 12 | 93 ± 6 | 19 ± 2 |
| | +DCA(2mM) | 189±29 | 85±15 | 16±4 |
| | Metoprolol (1 µM) | 185±40 | 73±17 | 13±6 |
| | +DCA (2 mM) | 193±20 | 87±10 | 7±3 |
| **LOW FLOW** | | | | |
| **ISCHEMIA: AEROBIC** | Control (0.05%DMSO) | 210 ± 5 | 158 ± 8 | 33 ± 1 |
| | DCA(2mM) | 214 ± 7 | 139 ± 3 | 30 ± 1 |
| | Diltiazem (0.8 µM) | 215 ± 6 | 131 ± 6 | 28 ± 1 |
| | +DCA(2mM) | 206 ± 6 | 147 ± 9 | 30 ± 2 |
| | Metoprolol (1 µM) | 216 ± 10 | 141 ± 7 | 30 ± 2 |
| | +DCA(2mM) | 227 ± 9 | 141 ± 8 | 32 ± 2 |
| **LOW FLOW** | | | | |
| **ISCHEMIA: ISCHEMIC** | Control (0.05%DMSO) | 210 ± 5 | 158 ± 8 | 33 ± 1 |
| | DCA(2mM) | 214 ± 7 | 139 ± 3 | 30 ± 1 |
| | Diltiazem (0.8 µM) | 215 ± 6 | 131 ± 6 | 28 ± 1 |
| | +DCA(2mM) | 206 ± 6 | 147 ± 9 | 30 ± 2 |
| | Metoprolol (1 µM) | 216 ± 10 | 141 ± 7 | 30 ± 2 |
| | +DCA(2mM) | 227 ± 9 | 141 ± 8 | 32 ± 2 |

**Table 2: Effect of various drugs on cardiac output, work and efficiency; aortic and coronary flow; and oxygen consumption.**

| | **Condition** | **Cardiac Output** | **Aortic Outflow** | **Coronary Flow** | **Cardiac Work** | **O₂ Consumption** | **Cardiac Efficiency** |
|---|---|---|---|---|---|---|---|
| | | **(ml•min⁻¹)** | **(ml•min⁻¹)** | **(ml•min⁻¹)** | **(CO•PSP)** | **(µmolO₂•g dry wt⁻¹•min⁻¹)** | **(CW•O₂⁻¹)** |
| **AEROBICALLY PERFUSED** | Control(0.05%DMSO) | 52±5 | 27±1 | 23±4 | 72±10 | 41±4 | 1,78±0.17 |
| | DCA(2mM) | 49±4 | 30+3 | 20±1 | *61±5* | 38±5 | 1.69±0.19 |
| | Diltiazem (0.8 µM) | 41±1 | 21±1 | 20±1 | 51±1 | 38±9 | 1.63±0.37 |
| | +DCA(2mM) | 46±2 | 26±2 | 21±1 | 61±6 | 30±5 | 2.17±0.25 |
| | Digoxin(3nM) | 52±1 | 31±1 | 21± 1 | 67±3 | 37±3 | 1.87±0.26 |
| | +DCA(2mM) | 43±1 | 25±2 | 18±1 | 53±1 | 41±4 | 1.30±0.11 |
| | Metoprolol(1µM) | 55±8 | 35±5 | 20±3 | 71±10 | 40±9 | 1.85±0.15 |
| | +DCA(2mM) | 43±1 | 25±2 | 19±1 | 56±3 | 33±4 | 1.74±0.22 |
| | Dobutamine(1µM) | 52±2 | 32±3 | 20±4 | 64±5 | 44±9 | 1.56±0.43 |
| | +DCA(2mM) | 55±4 | 32±3 | 23±1 | 72±8 | 60±5 | 1.24±0.17 |
| **GLOBAL ISCHEMIA** | Aerobic Control (0.05%DMSO) | 59±4 | 32±4 | 27±1 | * | * | * |
| | Post-Ischemic Control (0.05%DMSO) | 14±4 | 2±2 | 12±3 | * | * | * |
| | DCA(2mM) | 35±5 | 14±6 | 21±1 | * | * | * |
| | Digoxin(3nM) | 34±3 | 14±3 | 20±1 | * | * | * |
| | +DCA(2 mM) | 25±8 | 12±5 | 13±3 | * | * | * |
| | Metoprolol (1µM) | 24±9 | 11±5 | 13±4 | * | * | * |
| | +DCA (2 mM) | 24±3 | 8±4 | 16±2 | * | * | * |
| **LOW FLOW ISCHEMLA: AEROBIC** | **Control (0.05%DMSO)** | **49 ± 2** | **25 ± 2** | **24 ± 1** | ***** | ***** | ***** |
| | DCA(2 mM) | 50±2 | 27+3 | 22±1 | * | * | * |
| | Diltiazem (0.8 µM) | 43±1 | 22±1 | 21±1 | * | * | * |
| | +DCA (2 mM) | 51±1 | 30±2 | 22±1 | * | * | * |
| | Nletoprolol(1µM) | 47±2 | 25±2 | 22±1 | * | * | * |
| | +DCA (2 mM) | 58±2 | 34±3 | 23±1 | * | * | * |
| **LOW FLOW ISCHEMIA: ISCHEMIC** | Control (0.05%DMSO) | 26 ± 4 | 12± 5 | 14 ± 1 | * | * | * |
| | DCA(2 (2 mM) | mM) 26 ± 5 | 13±5 | 13 ± 1 | * | * | * |
| | Diltiazem (0.8 µM) | 24 ± 4 | 11±4 | 13 ± 1 | * | * | * |
| | +DCA (2 mM) | 36 ± 4 | 23 ± 3 | 13 ± 1 | * | * | * |
| | Metoprolol (1µM) | 31±4 | 17±3 | 14±1 | * | * | * |
| | +DCA(2 mM) | 37±4 | 22 ± 4 | 15±1 | * | * | * |

| | | | | | | | |
|---|---|---|---|---|---|---|---|
| * Data depicted graphically in Figures. | | | | | | | |

### EXAMPLE 2

### Effects of digoxin (3nM) and digoxin (3nM) with DCA (2mM) on glucose oxidation, glycolysis, cardiac function and efficiency in normal hearts.

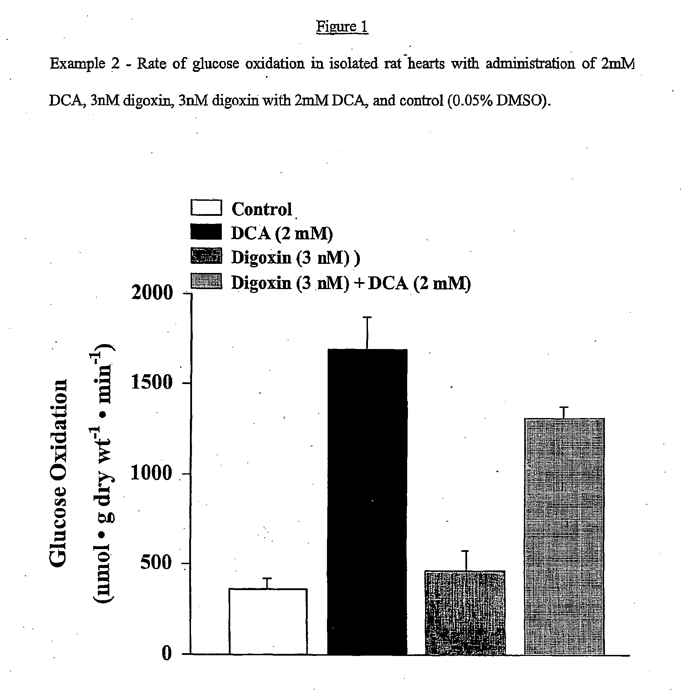

As shown in Figure 1, the Na⁺/K⁺ ATPase inhibitor digoxin, when compared to control, did not show a significant increase in glucose oxidation rates (361 ± 43 vs 469 ± 111 respectively). In addition, when compared to DCA treated hearts, digoxin appears to attenuate the stimulatory effects of DCA on glucose oxidation (1697± 179 vs 1314 ±62, respectively; Figure 1).

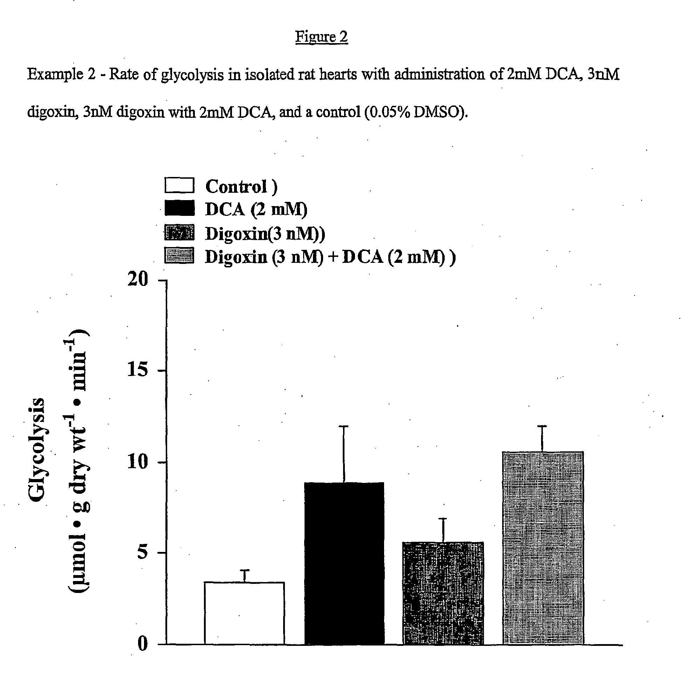

In Figure 2 DCA showed an increase in glycolysis when compared to control, but this increase was not significant (8.917 ± 3.060 vs. 3.430 ± 0.604, respectively). Digoxin alone increased glycolytic rates when compared to control (5.651 ± 1.298 vs. 3.430 ± 0.604, respectively; Figure 2). Digoxin with DCA increased glycolytic rates when compared to control rates (9.028, vs. 3.430 ± 0.604, respectively; Figure 2) and DCA alone (9.028 vs. 8.917 ± 3.060; Figure 2).

Digoxin had no significant effect on any of the functional parameters shown in Table 1. When combined with DCA, digoxin had a negative effect on peak systolic pressure when compared to control (121 ± 1 vs. 137 ± 5, respectively).

Table 2 shows that digoxin has a small but significant effect on aortic outflow when compared to control (31 ± 1 vs. 27 ± 1, respectively). Treatment with digoxin and DCA together returned the aortic out flow to control levels. However, when digoxin is used in conjunction with DCA, cardiac work is significantly reduced when compared to control (53 ± 1 vs. 72 ± 10, respectively). As well, when compared to digoxin treatment alone, digoxin and DCA together cause a significant decrease in cardiac work (67 ± 3 vs. 53 ± 1, respectively).

Although digoxin has no effect on cardiac metabolism, decreases in overall workload lead to a decrease in cardiac efficiency when digoxin is used along with DCA in normal hearts. As will be seen in later examples contained herein, this decrease in efficiency is clearly absent in hearts subjected to global ischemia, with clear benefit to overall workload observed in hearts treated with DCA and digoxin regardless of the decreased efficiency observed in aerobic models

### EXAMPLE 3

### Effects of diltiazem (0.8 µM) and diltiazem (0.8µM) with DCA (2mM) on glucose oxidation, glycolysis, cardiac function and efficiency in normal hearts.

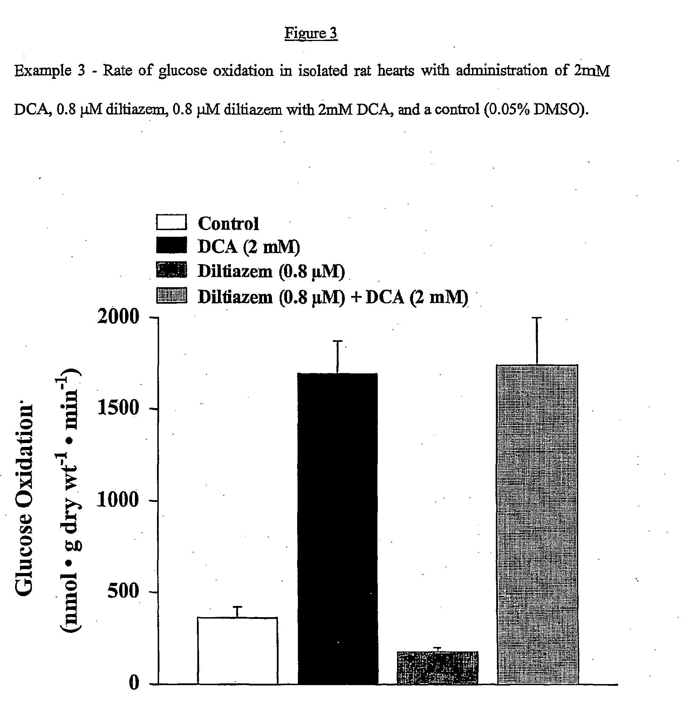

Diltiazem is a Ca²⁺ channel blocker. Figure 3 shows that when compared to control hearts, diltiazem caused a significant decrease in the rates of glucose oxidation (361 ± 43 vs 175:1 ± 24 respectively). When hearts were treated with diltiazem and DCA together, the effect of diltiazem alone on glucose oxidation was blocked and a significant increase in glucose oxidation was seen when compared to control (1737± 237 vs. 361 ±63; Figure 3). Though, this increase in glucose oxidation was no different than treating the hearts with DCA alone (1737 ± 264 vs. 1526 ± 79; Figure 3).

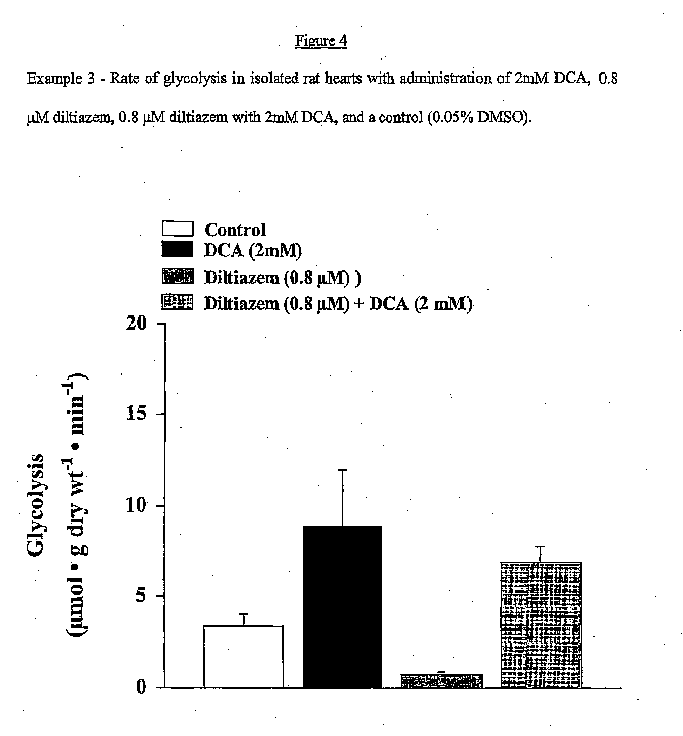

Diltiazem alone also had an effect on glycolytic rates when compared to control (0.727 ± 0.160 vs. 3.430 ± 0.604, respectively; Figure 4). Diltiazem and DCA together result in an attenuation of the effect of DCA alone (6.865 ± 0.887 vs. 8.917 ± 3.060, respectively; Figure 4). As well, DCA was able to overcome the attenuating effects of diltiazem.

Treatment with diltiazem caused a significant decrease in heart rate when compared to control (202 ± 3 vs. 239 ± 12, respectively; Table 1). As well, a significant decrease in HR x PSP was observed (25 ± 1 vs. 32 ± 1, respectively; Table 1). When treated with diltiazem and DCA, these functional parameters are returned to control levels (Table 1).

As shown in Table 2, diltiazem caused a significant decrease in aortic outflow when compared to controls (21 ± 1 vs 27 ± 1, respectively). Though significant, a decrease in cardiac work was observed when comparing diltiazem treated hearts to control hearts (51 ± 1 vs. 72 ± 10).

The calcium channel blocker diltiazem, has the ability to block the influx of Ca²⁺ into muscle cell and therefore prevents contraction. Diltiazem causes a reduced heart rate as well as a decrease in cardiac work. The overall effect of diltiazem is a reduction in the functional performance of the heart, resulting in a concommitment drop in metabolism. When hearts are treated with DCA and diltiazem together the inhibitory effects of diltiazem on metabolism are prevented and functional decreases seen with diltiazem are reversed.

### EXAMPLE 4

### Effects of dobutamine (1 µM) and dobutamine (1 µM) with DCA (2mM) on glucose oxidation, glycolysis, cardiac function and efficiency in normal hearts.

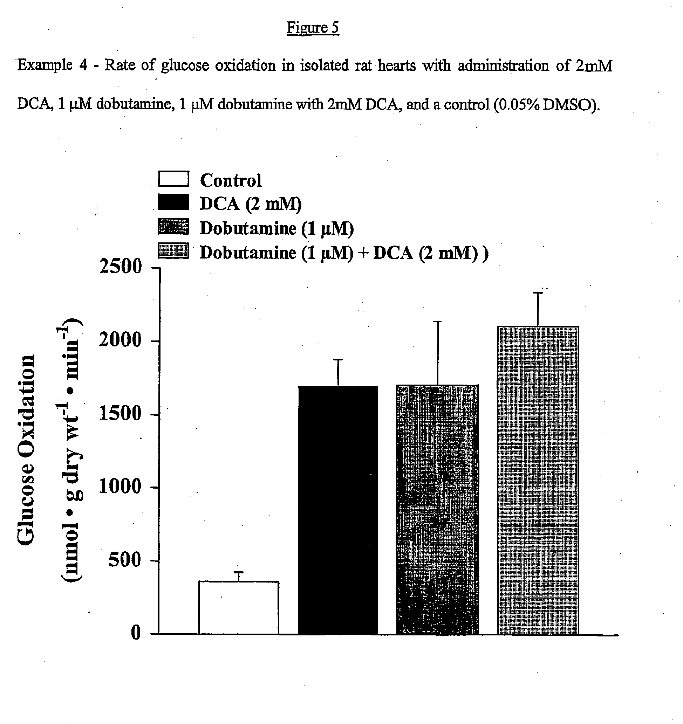

Dobutamine is a β₁-adrenoreceptor agonist (catecholamine). When compared to control, there is a large and significant increase in glucose oxidation (361 ± 43 vs 1707 ± 435, respectively; Figure 5). The effects of dobutamine on glucose oxidation mirrored that of DCA, but no significant additive effect was seen when the hearts were treated with DCA and dobutamine (1697 ± 179 vs. 2105 ± 232, respectively; Figure 5).

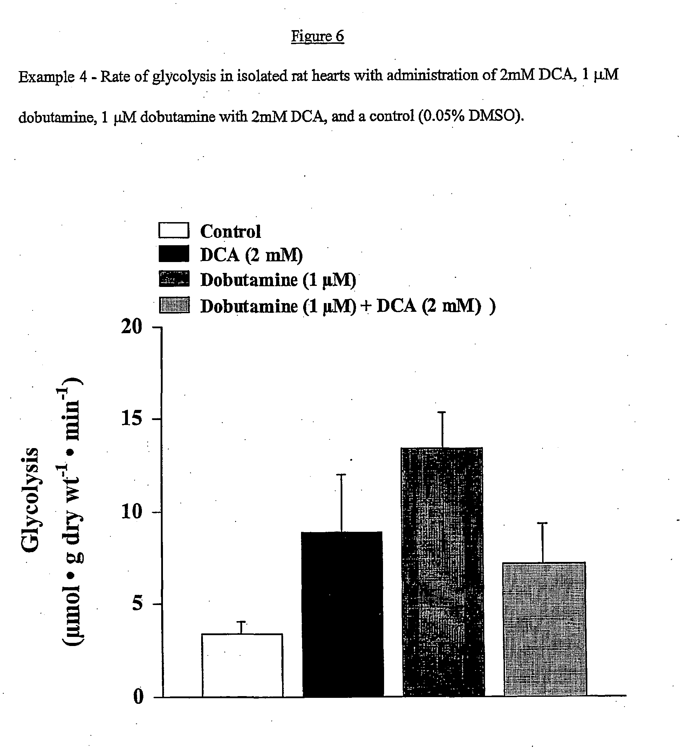

When compared to control, dobutamine treated hearts resulted in a large and significant increase in glycolysis (3.430 ± 0.604 vs. 13.365 ± 1.981, respectively Figure 6). The effects of dobutamine on glycolysis mirrored those of DCA, but no additive effect was seen when the hearts were treated with DCA and dobutamine (8.917 ± 3.060 vs. 7.187 ± 2.163, respectively; Figure 6).

In Table 1, dobutamine is shown to increase heart rate when compared to controls. (333 ± 7 vs. 239 ± 12, respectively). As well, HR x PSP increased significantly in the dobutamine treated group versus control (40 ± 2 vs. 32 ± 2, respectively). Treatment with DCA and dobutamine together, show no different effect on the functional parameters shown in Table 1, than treatment with dobutamine alone.

Table 2. shows that treatment with dobutamine alone has no significant effect on the functional parameters shown. Though, when dobutamine is used in conjunction with DCA, a significant increase in O₂ consumption is observed and a decrease in cardiac efficiency is observed as well.

While dobutamine was able to stimulate glucose oxidation it also increased glycolysis that would result in an increase in overall workload. In the presence of DCA and dobutamine, this uncoupling of glycolysis from glucose metabolism was attenuated and could lead to a decrease of H⁺ production and beneficial effect to the patient. Increased oxygen consumption results in a decrease in cardiac efficiency, though this is balanced with the observed increase in cardiac work.

### EXAMPLE 5

### Effects of metoprolol (1 µM) and metoprolol (1 µM) with DCA (2mM) on glucose oxidation, glycolysis, cardiac function and efficiency in normal hearts.

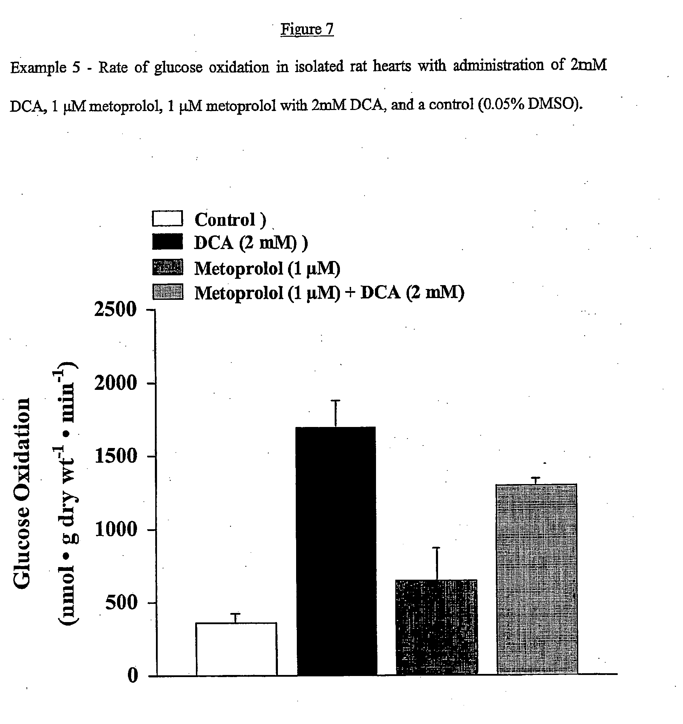

Metoprolol is a β₁-adrenoreceptor antagonist. Its effect on glucose oxidation, when compared to control, was insignificant (361 ± 43 vs. 651 ± 222 respectively; Figure 7). When combined with DCA, a trend was seen towards the attenuation of the effects of DCA on glucose oxidation, but this trend was not significant (1297 ± 40 vs. 1697 ± 179, respectively; Figure 7).

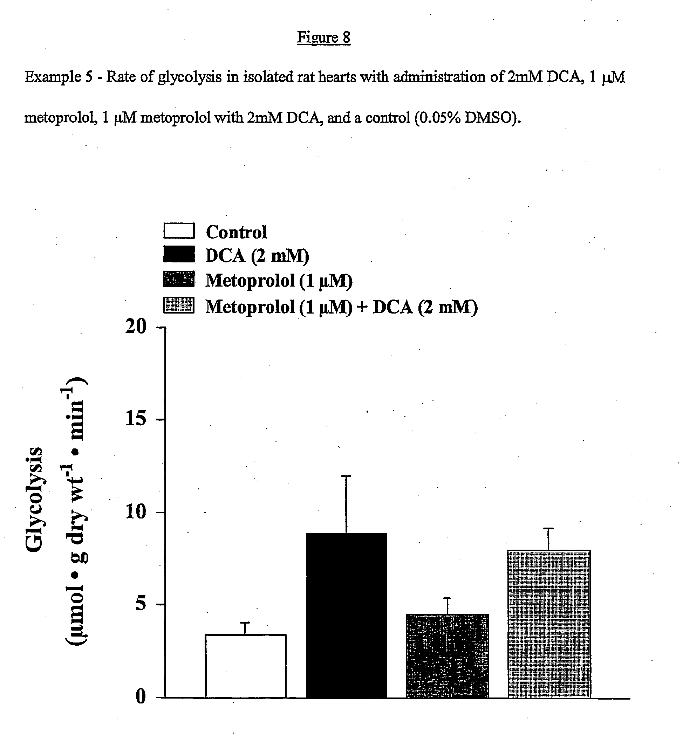

The effect of metoprolol on glycolysis when compared to control was insignificant (3.430 ± 0.604 vs. 4.530 ± 0.876 respectively; Figure 8). When combined with DCA, there was also no change when comparing DCA treatment and DCA with metoprolol (8.917 ± 3.060 vs. 8.022 ± 1.132 respectively; Figure 8)

Table 1 and Table 2 show that metoprolol had no significant effect on any of the functional parameters shown. However, when metoprolol is used in combination with DCA, coronary flow and cardiac work significantly reduced.

### EXAMPLE 6

### Effects of digoxin (3nM) and digoxin (3nM) with DCA (2mN on glucose oxidation, glycolysis, cardiac function and efficiency in hearts subjected to global ischemia.

The effects of the Na+/K+ ATPase inhibitor digoxin in combination with DCA on post-ischemic hearts was determined. As shown in Table 1 and Table 2, all functional attributes measured are depressed during the reperfusion period when compared to the pre-ischemic aerobic values. However when compared to the control values during the reperfusion period, the addition of DCA, digoxin, or digoxin with DCA appear to significantly improve both cardiac function and cardiac efficiency. When compared to the control values during the reperfusion period, the addition of DCA, digoxin, or digoxin with DCA appears to significantly improve cardiac function and cardiac efficiency.

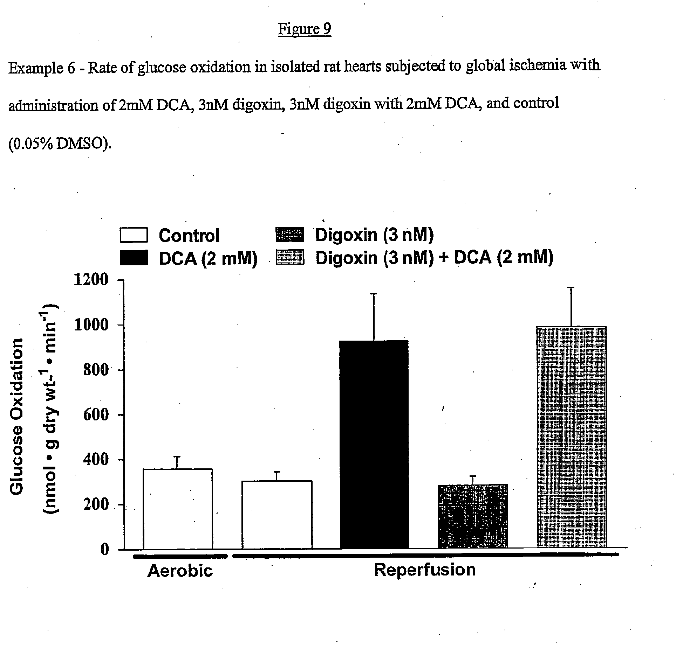

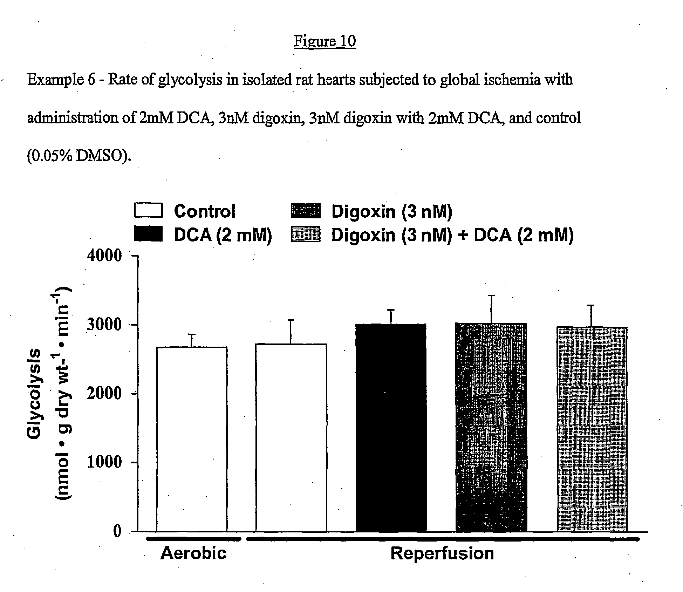

As shown in Figure 9, DCA appears to have significantly higher glucose oxidation rates when compared to either the aerobic control, reperfused control, or digoxin alone. The combination of digoxin and DCA appears to enhance the ability of DCA to improve glucose oxidation rates and is significantly higher than DCA alone. As shown in Figure 10, neither DCA, digoxin, nor digoxin with DCA altered glycolytic rates as compared to control.

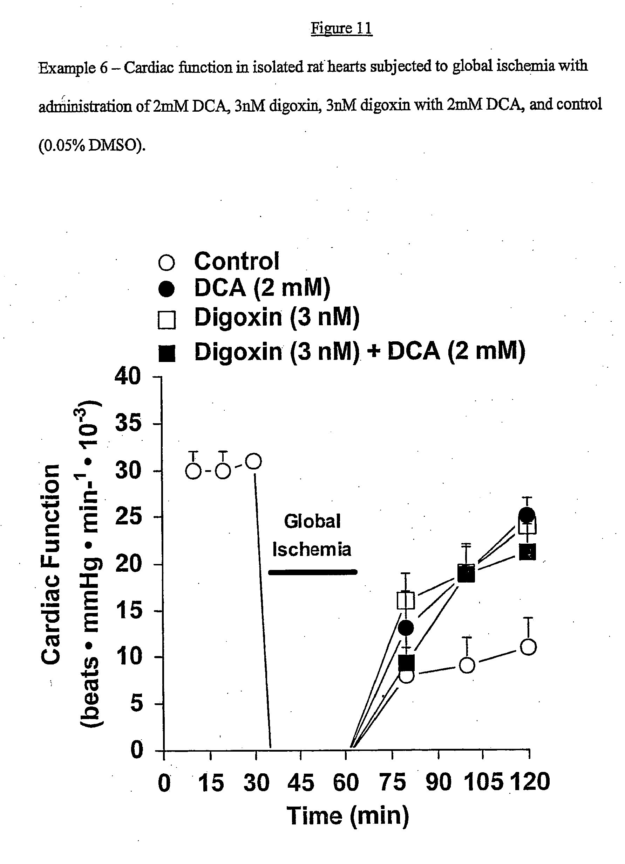

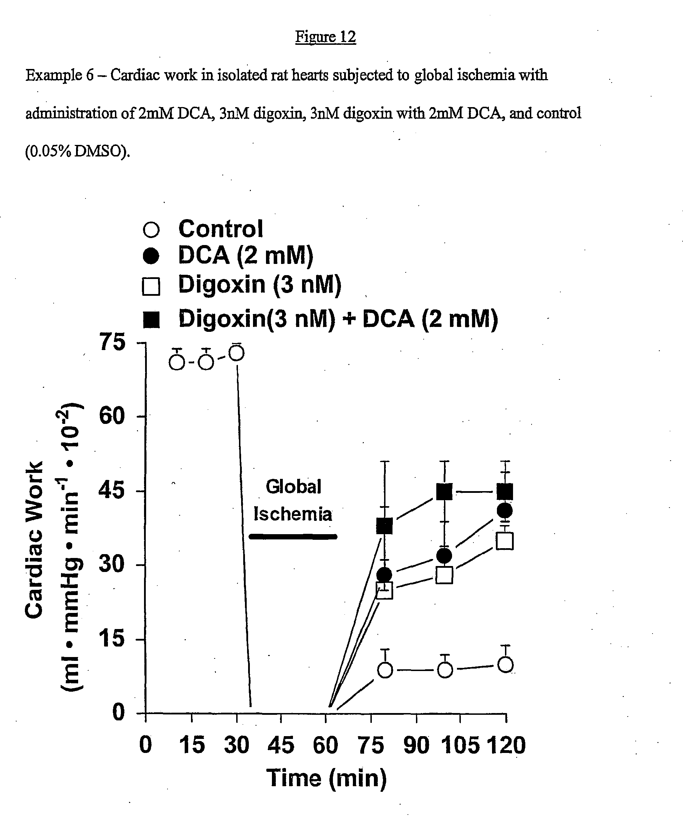

As shown in Figure 11, DCA, digoxin, or digoxin with DCA significantly improves the recovery of cardiac function of previously ischemic hearts, when compared to the recovery of control hearts during the reperfusion period. As shown in Figure 12, DCA, digoxin, or digoxin with DCA appear to significantly improve the recovery of cardiac work of previously ischemic hearts when compared to the recovery of control hearts during the reperfusion period.

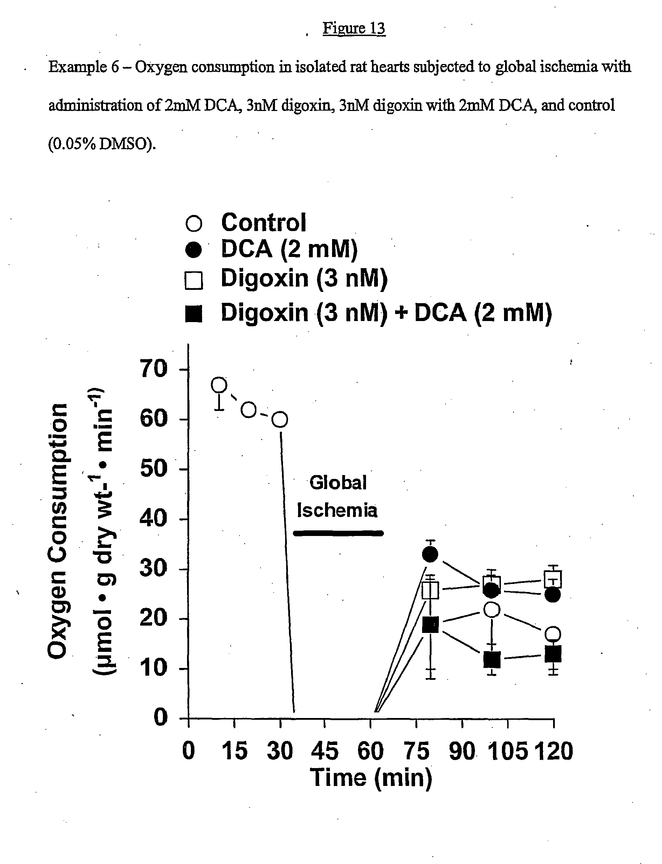

As shown in Figure 13, DCA, digoxin, or digoxin with DCA significantly improve the recovery of oxygen consumption of previously ischemic hearts when compared to the recovery of control hearts during the reperfusion period.

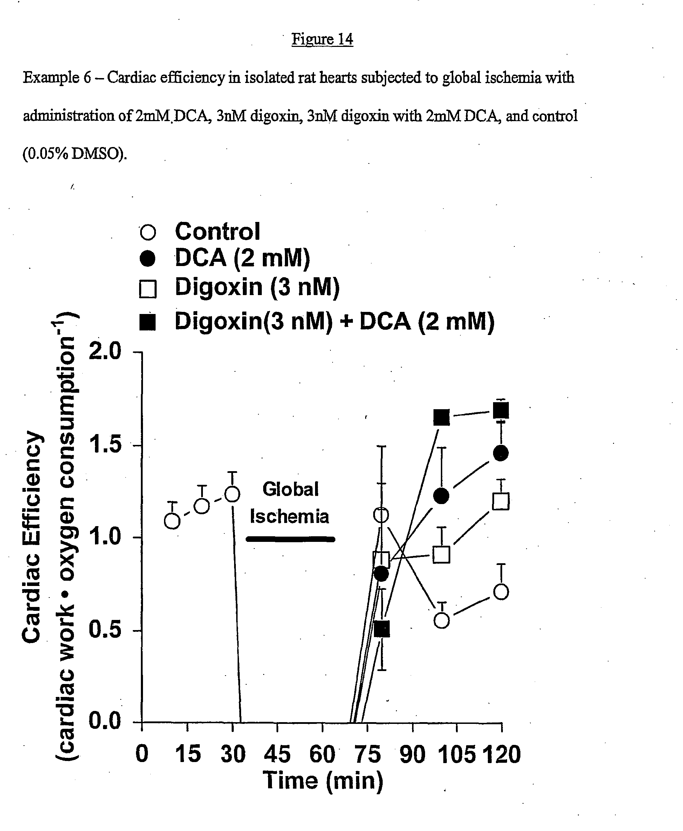

As shown in Figure 14, DCA, digoxin, or digoxin with DCA significantly improve the recovery of cardiac efficiency of previously ischemic hearts, when compared to the recovery of control hearts during the reperfusion period.

### EXAMPLE 7

### Effects of metoprolol (1 µM) and metoprolol (1 µM) with DCA (2mM) on glucose oxidation, glycolysis, cardiac function and efficiency in hearts subjected to global ischemia.

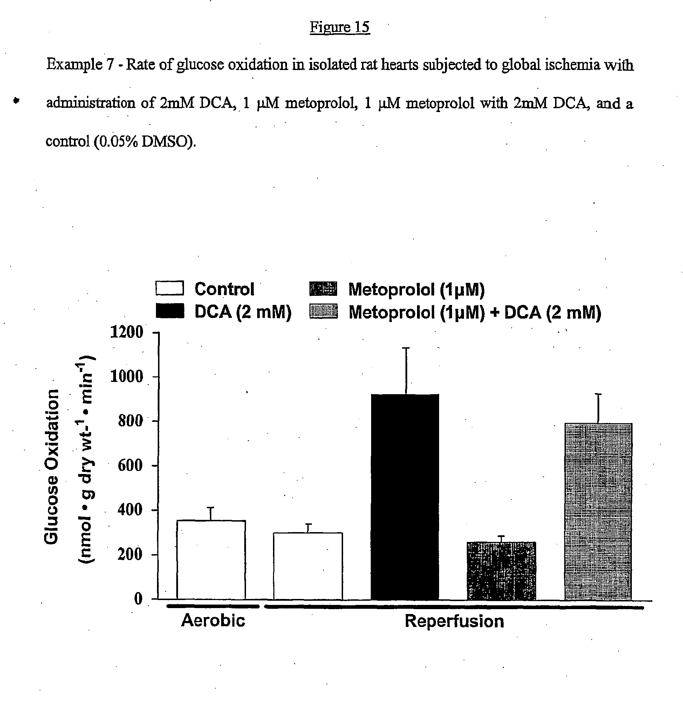

The β₁-adrenoreceptor antagonist metoprolol and metoprolol with DCA significantly improved functional parameters of the hearts during the reperfusion period, compared to control (Table 1, Table 2). As shown in Figure 15, the metoprolol had no effect on glucose oxidation rates during the reperfusion period.

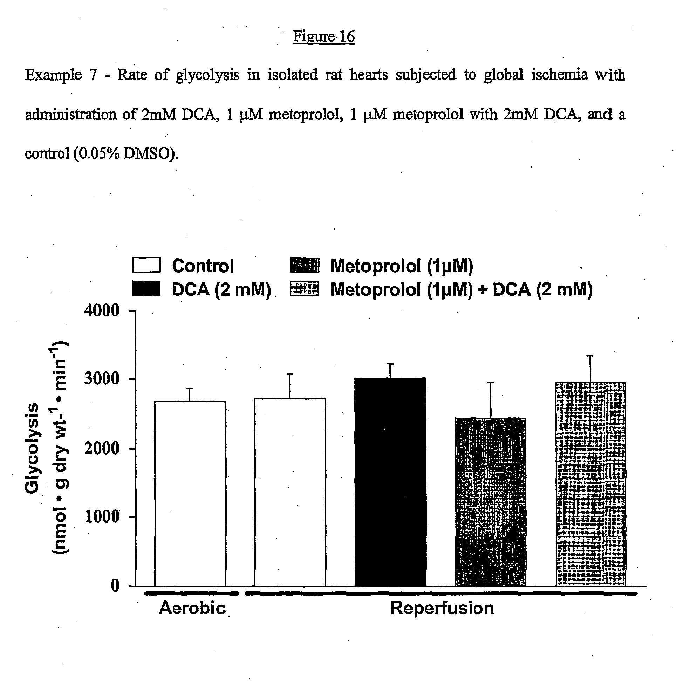

DCA or the combination of metoprolol with DCA appeared to have significantly increased glucose oxidation rates when compared to either metoprolol or control during both the aerobic and reperfusion periods. As can be seen in Figure 16, neither DCA, metoprolol or metoprolol with DCA altered glycolytic rates as compared to control

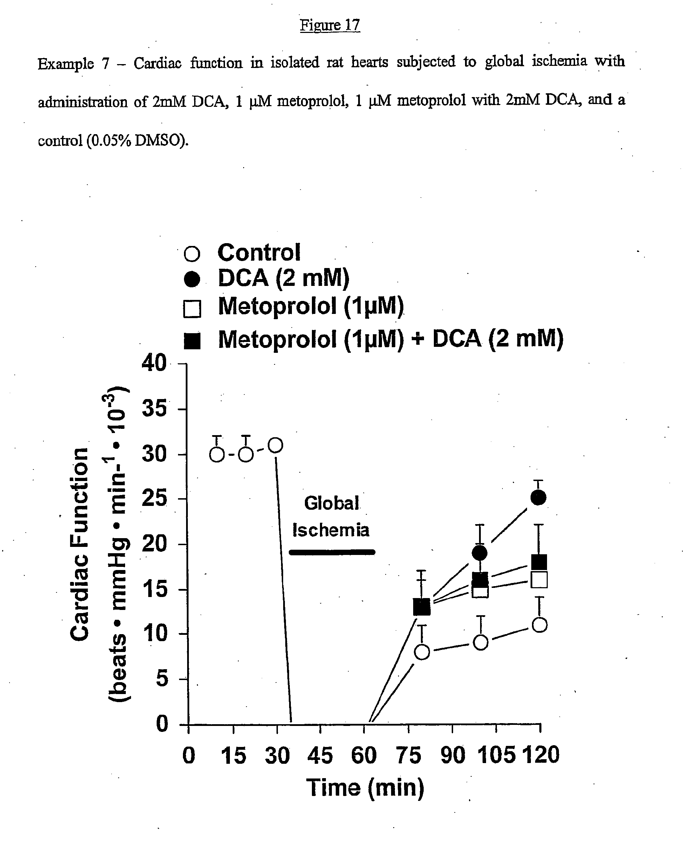

As shown in Figure 17, DCA appears to significantly improve the recovery of cardiac function of previously ischemic hearts when compared to the recovery of control hearts during the reperfusion period. However, metoprolol or the combination of metoprolol with DCA does not change cardiac function as compared to control.

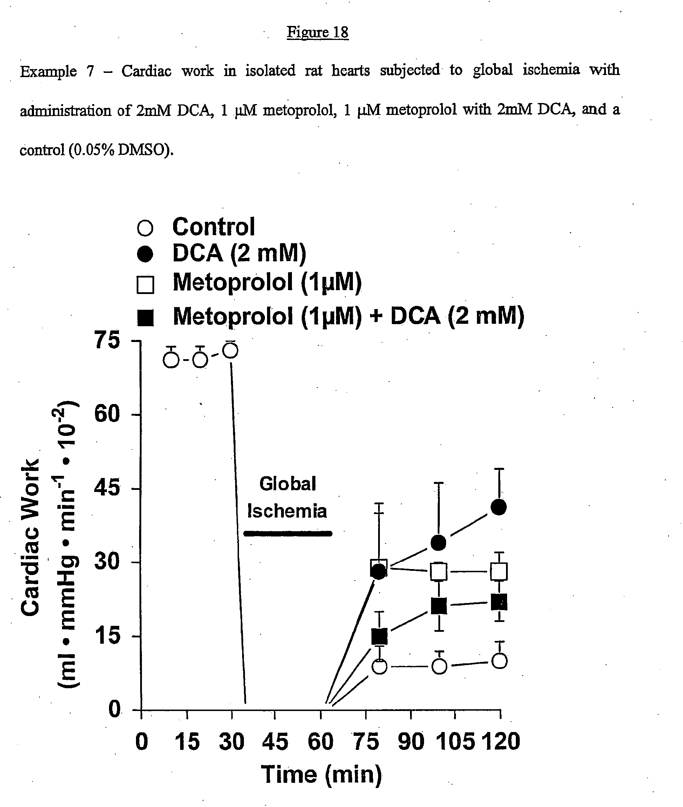

As shown in Figure 18, DCA, metoprolol, or metoprolol with DCA appear to significantly improve the recovery of cardiac work of previously ischemic hearts when compared to the recovery of control hearts during the reperfusion period. However the combination of DCA with metoprolol did not show an additive effect when compared to DCA alone.

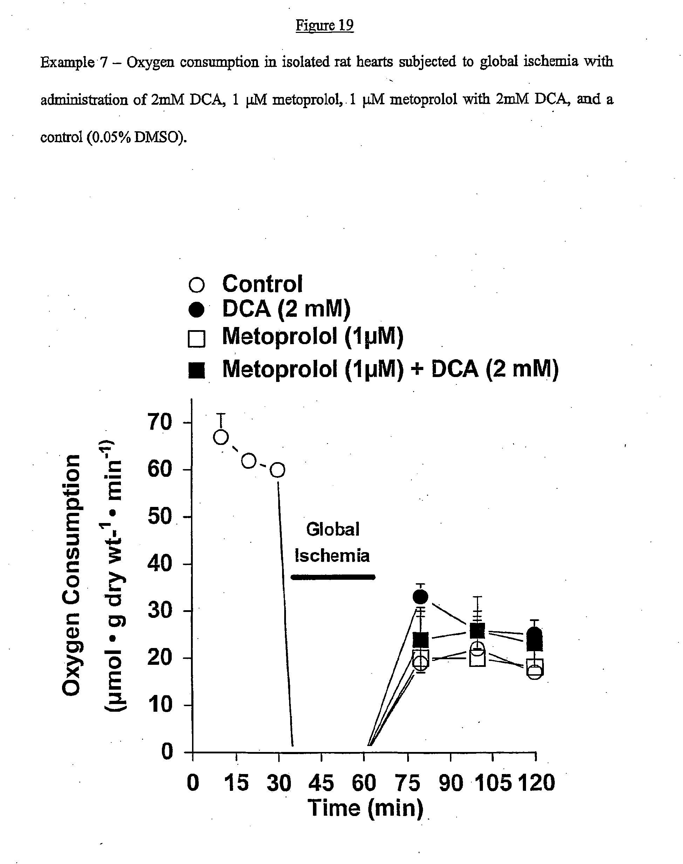

There does not appear to be significant differences with respect to recovery of oxygen consumption of previously ischemic hearts between DCA, metoprolol, or metoprolol with DCA (Figure 19).

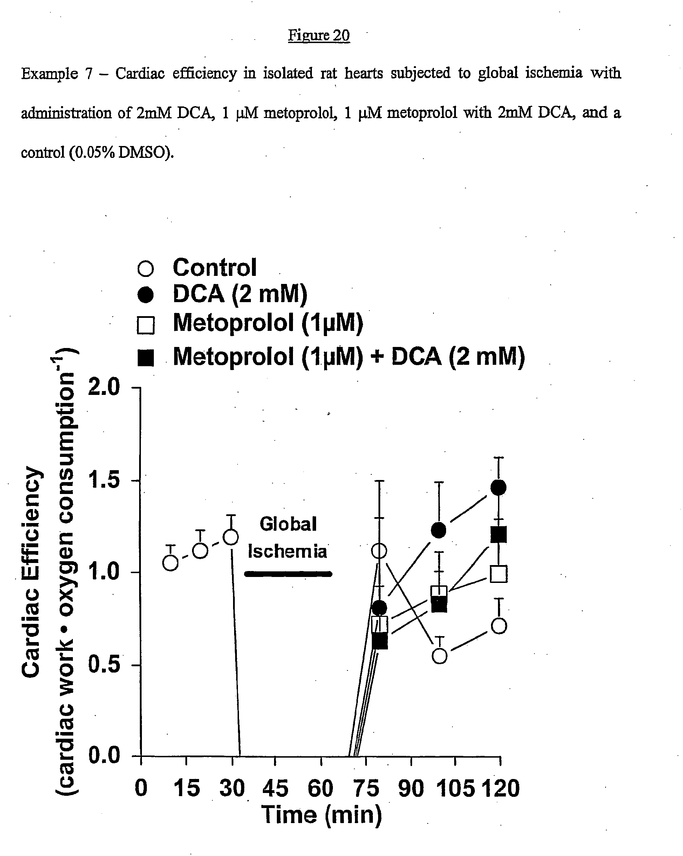

As shown in Figure 20, DCA or metoprolol with DCA appear to significantly improve the recovery of cardiac efficiency of previously ischemic hearts when compared to the recovery of control hearts during the reperfusion period. However, metoprolol alone does not As well the combination of DCA with metoprolol did not show an additive effect when compared to DCA alone.

### EXAMPLES 8

### Effects of diltiazem (0.8 µM) and diltiazeni (0.8µM) with DCA (2mM) on glucose oxidation, glycolysis, cardiac function and efficiency in hearts subjected to low flow demand ischemia.

As shown in Table 1, the presence of diltiazem decreased peak systolic pressure as compared to control. DCA with diltiazem increased peak systolic pressure as compared to DCA, diltiazem, or DCA with diltiazem (Table 1). Similar increases were observed for DCA with diltiazem in cardiac output, aortic outflow and coronary flow as compared to control and DCA or diltiazem alone (Table 2).

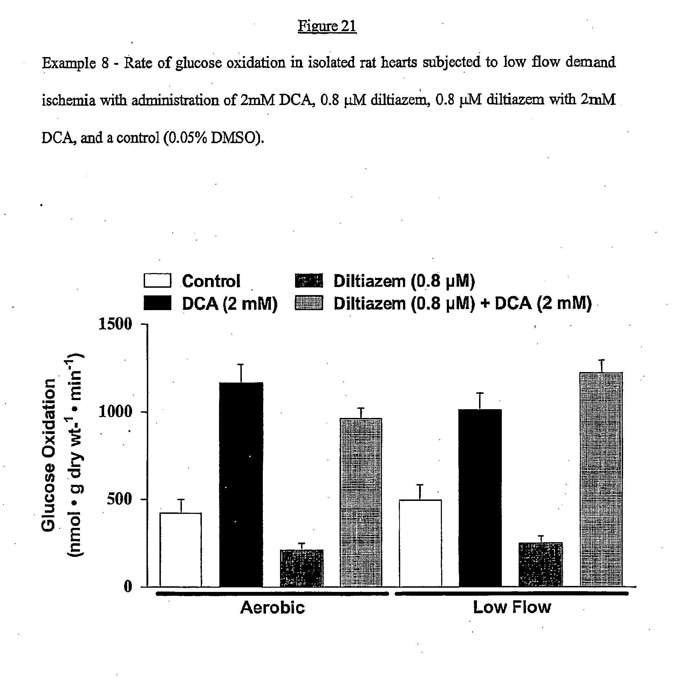

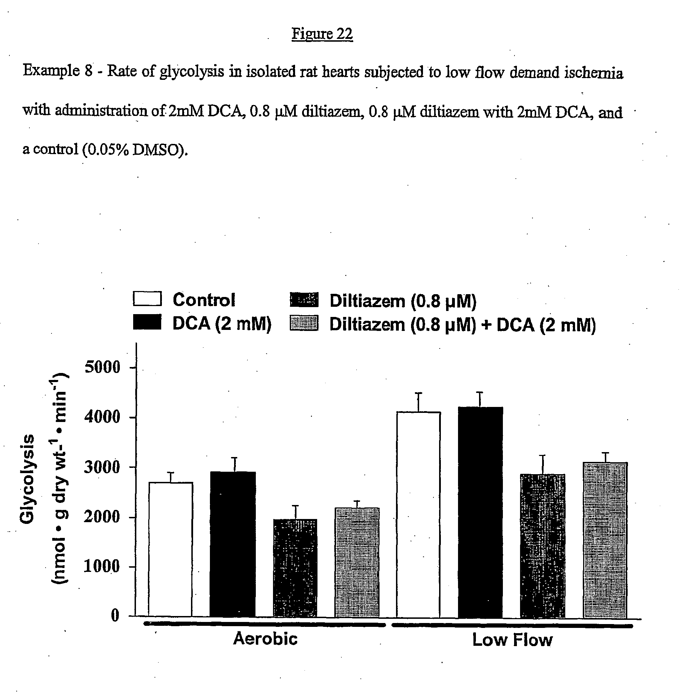

As shown in Figure 21, diltiazem did not appear to interfere with the ability of DCA to increase glucose oxidation in either the aerobic or low-flow ischemic perfusions. There did appear to be some attenuation of glycolysis by diltiazem alone, or with DCA in both aerobic and low-flow ischemic perfusions (Figure 22).

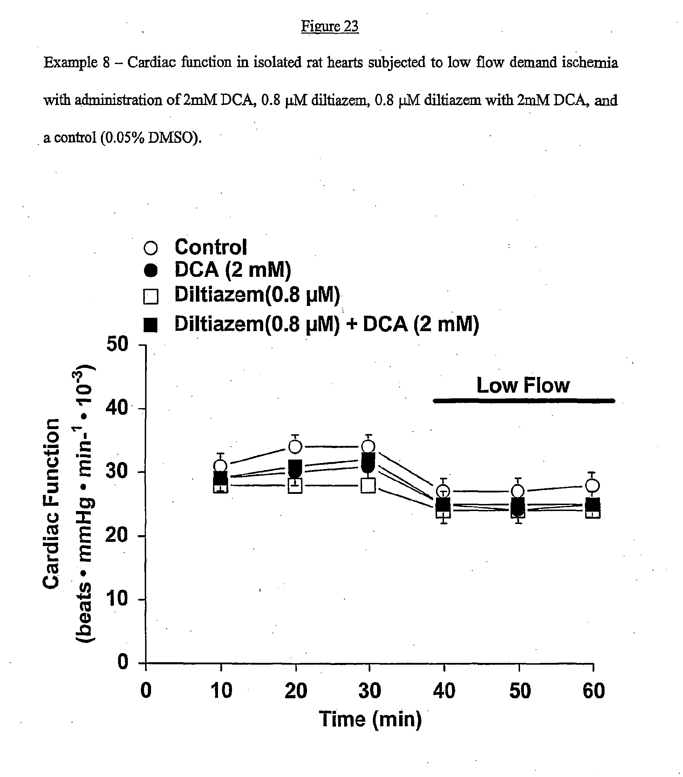

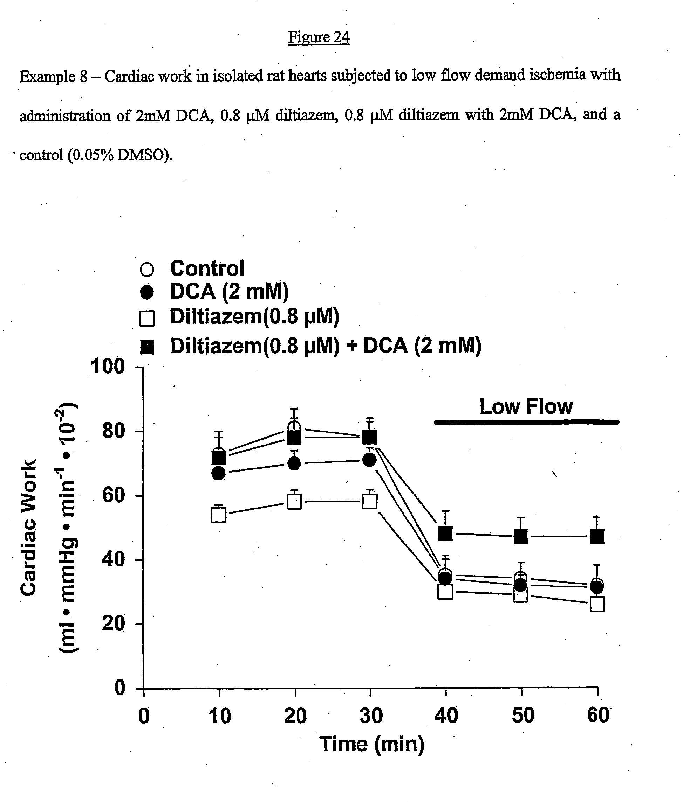

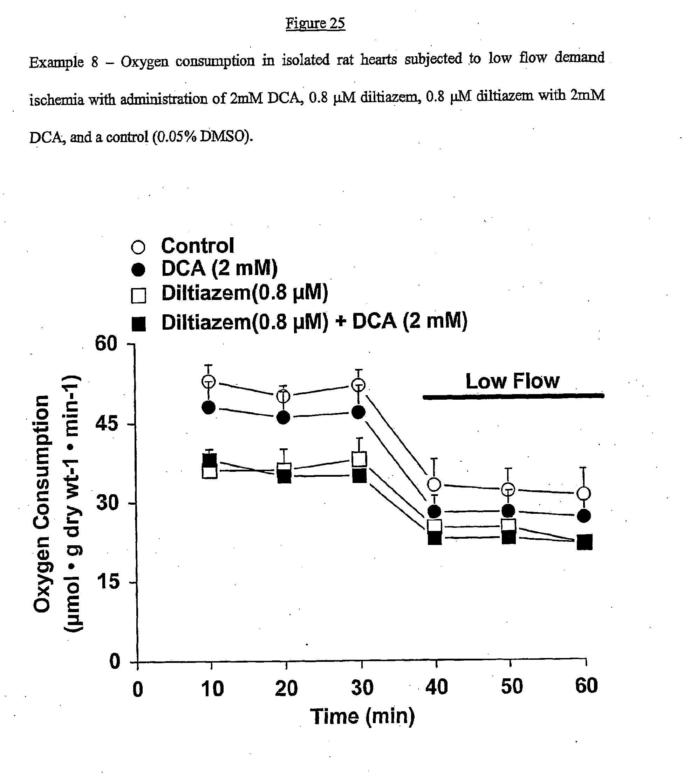

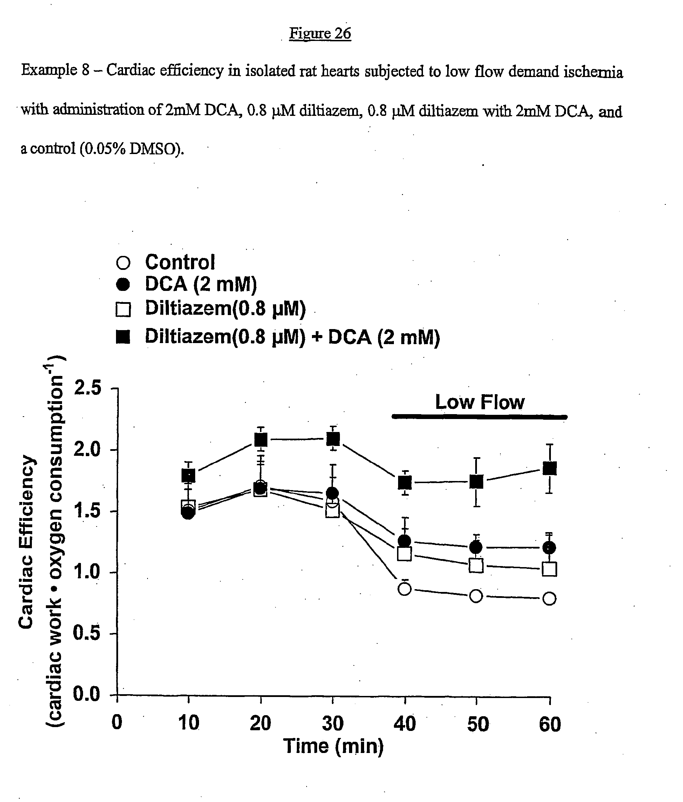

Neither diltiazem, DCA, nor DCA with diltiazem significantly affected cardiac function in the low-flow ischemic period (Figure 23). DCA with diltiazem significantly increased cardiac work (Figure 24) and decreased- oxygen consumption (Figure 25); thereby significantly increasing cardiac efficiency (Figure 26) compared to control, DCA and diltiazem alone.

### EXAMPLE 9

### Effects of metoprolol (1 µM) and metoprolol (1 µM) with DCA (2mM) on glucose oxidation, glycolysis, cardiac function and efficiency in hearts subjected to low flow demand ischemia.

As shown in Table 2, the addition of metoprolol with DCA caused a significant increase in aortic outflow and cardiac output as compared to control, metoprolol, or DCA alone. Compared to control there was no significant increase observed in coronary outflow (Table 2), heart rate (Table 1) or peak systolic pressure (Table 1) following the addition of DCA, metoprolol, or DCA with metoprolol. The decrease observed in peak systolic pressure observed in DCA and metoprolol was not compounded by simultaneous addition of the compounds together (Table 1).

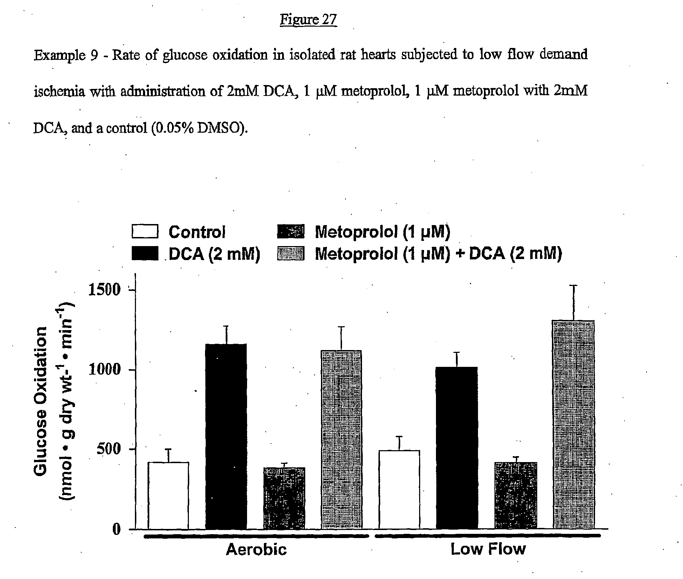

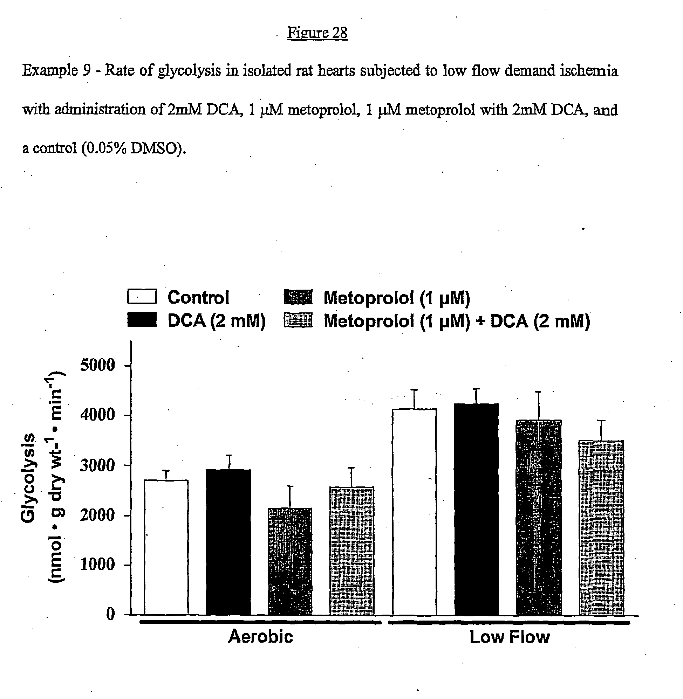

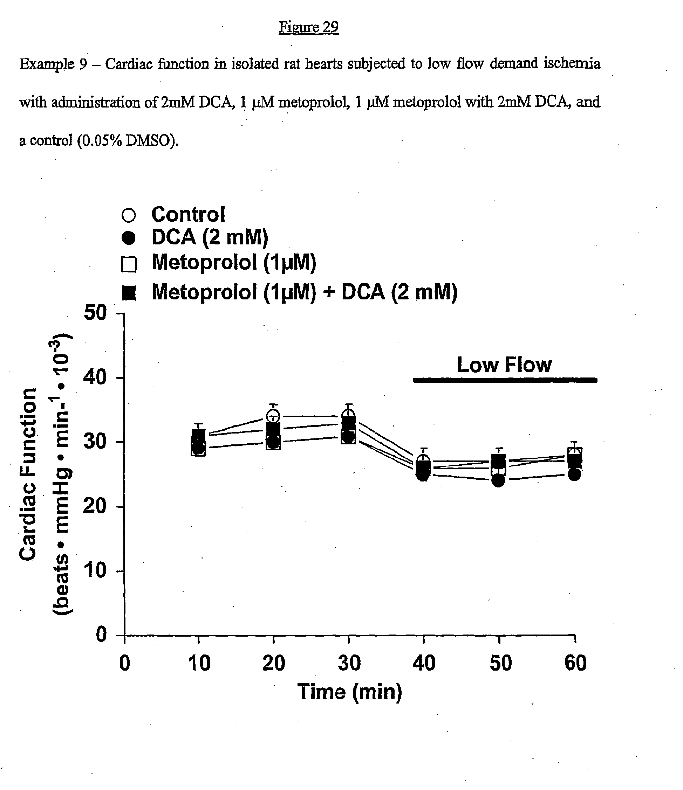

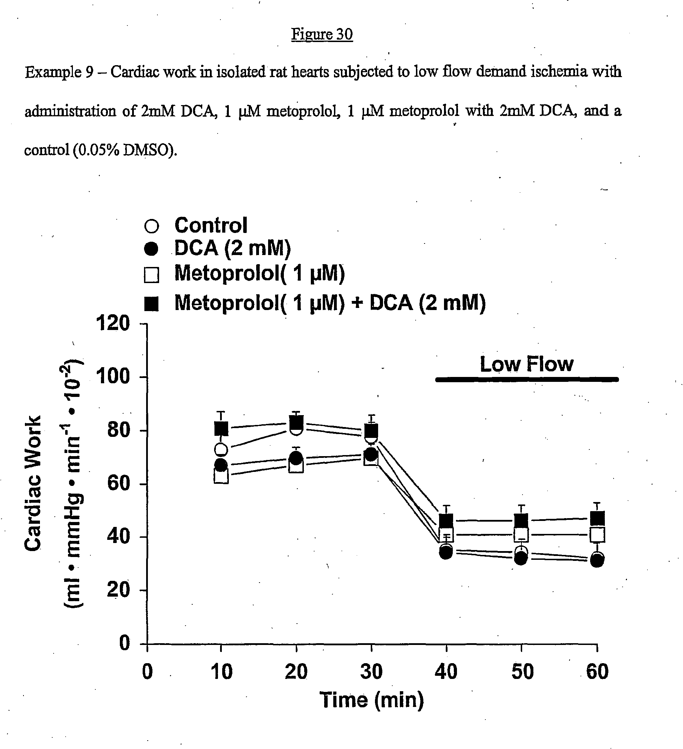

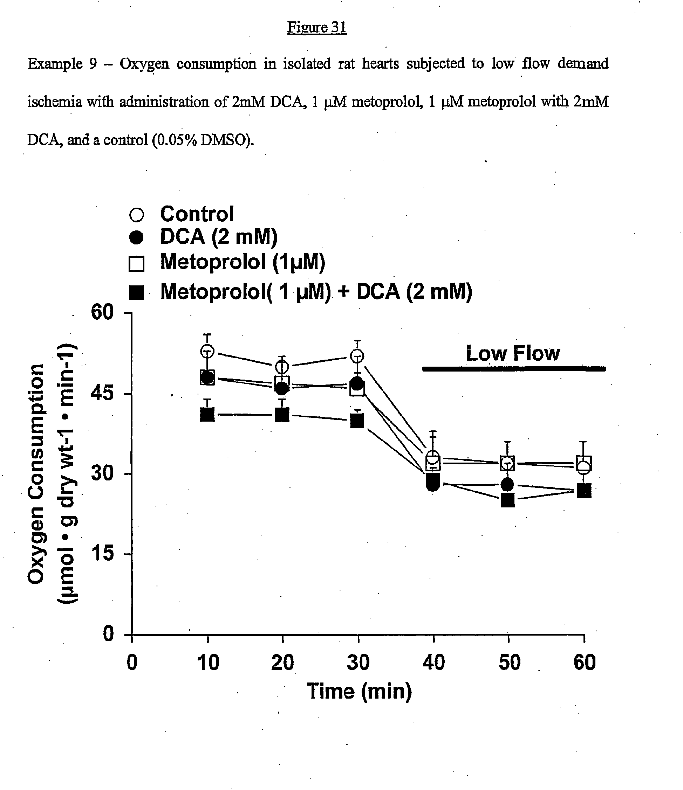

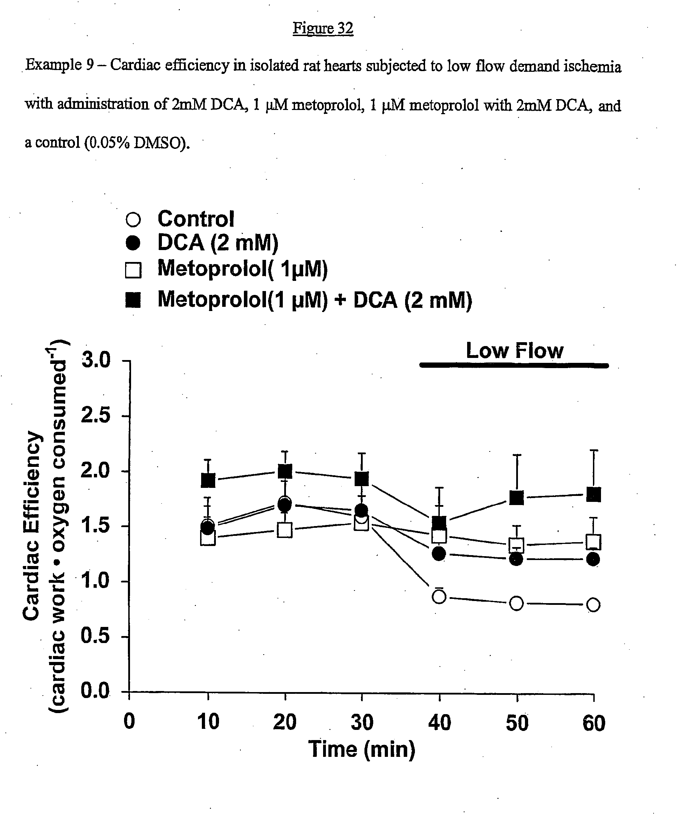

The presence of metoprolol does not appear to attenuate the increase in glucose oxidation observed with DCA alone (Figure 27) either in low-flow ischemic or aerobic conditions, nor are there significant changes observed to glycolysis in low-flow ischemic conditions (Figure 28). There are no significant changes to cardiac function (Figure 29), cardiac work (Figure 30), or oxygen consumption observed with addition of DCA, metoprolol, or DCA with metoprolol; as compared to control (Figure 31); though there is an observed increase in cardiac efficiency for DCA with metoprolol during low-flow ischemic conditions as compared to control (Figure 32).

### Conclusion

Metabolic modulators, such as DCA, can be used to shift the metabolism of the heart away from fatty acids oxidation and towards glucose oxidation. This shift has been shown to be beneficial during periods of cardiac stress such as angina, myocardial infarction or post-cardiac surgery. We sought to determine if the metabolic effects of using DCA are maintained in the presence of other cardioprotective or hemodynamic classes of drugs such as inotropic drugs (including digoxin and dobutamine), beta-blockers and calcium channel blockers. Isolated perfused working rat hearts were perfused in the presence of 5.5 mM glucose and 1.2 mM palmitate. Glucose oxidation and glycolysis were measured using [5-³H/U-14-¹⁴C] glucose.

In the aerobic model, the addition of DCA resulted in an increase in glucose oxidation of over 400%, while glycolysis increased over 300%. Digoxin and metoprolol showed no additive metabolic effect when used with DCA, nor did they have any metabolic effect when used alone. Diltiazem caused glucose metabolism to decrease when compared to control. DCA was able to overcome this effect when used with diltiazem. Dobutamine was able to increase glucose metabolism by almost 400%, but had no synergistic effect when combined with DCA. When used in conjunction with DCA neither digoxin, dobutamine, metoprolol, nor diltiazem were able to increase the effect that DCA alone had on metabolism. In addition, digoxin, metoprolol, diltiazem and dobutamine did not have any adverse effects on DCA's ability to increase glucose oxidation or glycolysis.

In aerobic perfusions, digoxin in combination with DCA has been shown to lower overall cardiac work with concomitant drop in cardiac efficiency. However, in our ischemia/reperfusion model, which mimics an ischemic event such as myocardial infarction and reperfusion, the combination of digoxin and DCA proved to be significantly beneficial as a synergistic combination to the recovery of cardiac work, cardiac efficiency and glucose oxidation. This suggests that digoxin in combination with DCA could be beneficial in post myocardial infarction followed by reperfusion or percutaneous coronary intervention (angioplasty) or during cardiac bypass surgery and open heart surgical procedures.

In aerobically perfused hearts, diltiazem causes an overall decrease in cardiac work and glucose metabolism. In our low flow ischemia model (which mimics angina), diltiazem has the same effect. This decrease in cardiac work with a maintenance of oxygen consumption led to a decrease in overall cardiac efficiency, both during the aerobic period and during low flow ischemia. However, the combination of diltiazem and DCA led to a reversal of these functional and metabolic decreases with a significant improvement in cardiac work, cardiac efficiency and glucose oxidation. As well, the combination of DCA and diltiazem is synergistic in combination and is more efficacious than DCA alone, with respect to maintenance of cardiac efficiency during low flow ischemia events such as angina.

As seen in the aerobic model, metoprolol alone has no effect on glucose oxidation, nor does it alter any functional parameters in aerobically perfused hearts. While metoprolol alone has a beneficial effect on recovery of previously ischemic hearts, the combination of metoprolol and DCA significantly improves recovery beyond that of the control hearts and beyond that of metoprolol alone, with respect to cardiac function. Using our low flow ischemia model, DCA and metoprolol appear to show a marked improvement in cardiac efficiency when compared to control during the low flow ischemic period. In addition, the combination of metoprolol and DCA significantly improve cardiac efficiency above that of control and DCA or metoprolol alone during the low flow ischemic period. This suggests that metoprolol with DCA is synergistic with respect to improving cardiac efficiency in treating ischemic conditions such as post myocardial infarction and heart failure.

Taken together, this data shows that DCA is able to significantly increase glucose metabolism and maintain function the presence of dobutamine, digoxin, diltiazem and metoprolol; thereby ameliorating the negative side effects of these drugs and drug classes. Furthermore the data indicates that DCA is synergistic with metoprolol, diltiazem and digoxin with respect to improving cardiac efficiency.

### References:

1 Lopaschuk G.D., Wambolt R.B., Harr R.L. An imbalance between glycolysis and glucose oxidation is a possible explanation for the detrimental effects of high levels of fatty acids during aerobic reperfusion of ischemic hearts. (1993) J Pharmacol Exp Ther. 264: 135-144.
2 Lopaschuk G.D., Belke D.D., Gamble J., Itoi T., Schonekess B.O. Regulation of fatty acid oxidation in the mammalian heart in health and disease. (1994) Biochim Biophys Acta. 1213:263-276.
3 Allard M.F., Schonekess HO, Henning SL, English DR, Lopaschuk GD. Contribution of oxidative metabolism and glycolysis to ATP production in hypertrophied hearts. (1994) Am J Physiol 267:H742-H750.
4 Mjos O.D., Miller N.E., Riemersma RA., Oliver M.F. Effects of dichloroacetate on myocardial substrate extraction, epicardial ST-segment elevation and ventricular blood flow following coronary occlusion in dogs. (1976) Cardiovasc Res 10:427-436.
5 Thannkikkotu B., Koshal A., Finegan B., Taylor D., Teo K., Chen R., Robertson M., Gunther C., Lopaschuk G.D. Dichloroacetate (DCA) stimulates pyruvate dehydrogenase complex (PDC) activity in hearts of patients undergoing coronary artery bypass grafting (CABG). (1994)Can. J. Cardiol. 10 (suppl. C): 130C.
6 Collins-Nakai R., Suarez-Almazor M., Karmy-Jones R., Penkoske P.A., Teo K., Lopaschuk G.D. Dichloroacetic acid (DCA) after open heart surgery in infants and children. (1995) Can. J. Cardiol. 11 (suppl. E):106E.
7 Cross, H.R. Trimetazidine for stable angina pectoris. (2001) Exp. Opin. Pharmacother. 2:857-875.
8 Bersin, R.M., Wolfe, C., Kwasman, M., Lau, D., Klinski, C., Tanaka, K., Khorrami, P., Henderson, G.N., DeMarco, T., Chatterjee, K., Improved Hemodynamic Function and Mechanical Efficiency in congestive Heart Failure with Sodium Dichloroacetate (1994) JACC 23(7):1617-1624.
9 Chandler, B.M., Sonnenblick, E.H., Pool, F.E. Mechanochemisty of Cardiac Muscle III. Effects of norepinephrine on the utilization of high energy phosphates. Circ. Res. (1968) 22:729-735.
10 Suga H., Hisano, R., Goto, Y., Yamada, O., Igarashi, Y. Effect of positive inotropic agents on the relation between oxygen consumption and systolic pressure volume area in canine left ventricles. (1983) Circ Res. 53:306-318.
11 Hasenfuss, G., Mulieri, L.A., Allen, P.D., Just, H., Alpert, N.R. Influence of isoproterenol and ouabin on excitation-contraction coupling, crossbridge function and energetics in failing human myocardium. (1996) 94:3155-3160.
12 Bing, R.J., Siegel, A., Vitale, A., Balboni, F., Sparks, E., Taeschler, M., Klapper, M., Edwards, W.S. Metabolic studies on the human heart in vivo. Studies on carbohydrate metabolism of the human heart. (1953) Am. J. Med. 15:284-296.
13 Lopaschuk, G.D., Wall, S.R., Olley, P.M., Davies, N.J. Etomoxir, a carnitine palmitoyltransferase I inhibitor, protects hearts from fatty acid-induced ischemic injury independent of changes in long chain acylarnitine. (1988) Circ. Res. 63(6): 1036-1043.
14 Lopaschuk, G.D., Spafford, M.A., Davies, N.J., Wall, S.R. Glucose and palmitate oxidation in isolated working rat hearts reperfused after a period of transient global ischemia. (1990) Circ. Res. 66:546-553.
15 Lopaschuk, G.D., Collins-Nakai, R., Olley, P.M., Montague, T.J., McNeil, G., Gayle, M., Penkoske, P., Finegan, B.A. Plasma fatty acid levels in infants and adults following myocardial ischemia. (1994) Am. Heart J., 128:61-67.
16. Saddik, M., Lopaschuk, G.D. Myocardial triglyceride turnover and contribution to energy substrate utilization in isolated working rat hearts. (1991) J. Biol. Chem. 266:8162-8170:
17 McVeigh, J.J., Lopaschuk, G.D. Dichloroactetate stimulation of glucose oxidation improves recovery of ischemic rat hearts. (1990) Am. J. Physiol. 259:H1079-1085.
18 Lopaschuk G.D., Wambolt R.B., Harr R.L. An imbalance between glycolysis and glucose oxidation is a possible explanation for the detrimental effects of high levels of fatty acids during aerobic reperfusion of ischemic hearts. (1993) J Pharmacol Exp Ther. 264:135-144.
19 Saddik M., Lopaschuk G.D.. Myocardial triglyceride turnover during reperfusion of isolated rat hearts subjected to a transient period of global ischemia. (1992) J Biol. Chem. 267:3825-3831.
20 Liu H., el Alaoui-Talibi Z., Clanachan A.S., Schulz R, Lopaschuk G.D. Uncoupling of contractile function from mitochondrial TCA cycle activity and MVO2 during reperfusion of ischemic hearts. (1996) Am J Physiol. 270:H72-H80.
21 Taegtmeyer H., Goodwin G.W., Doenst T., Frazier O.H. Substrate metabolism as a Determinant for Postischemic Functional Recovery of the heart. (1997) Am J Cardiol. 80:3A-10A.
22 Barbour R.L., Sotak C.H., Levy G.C., Chan S.H. Use of gated perfusion to study early effects of anoxia on cardiac energy metabolism: a new 31pNMR method. (1984) Biochemistr. 1923:6503-6062.

The invention furthermore comprises the following items;
1. A composition comprising a unit dosage form of dichloroacetate (DCA) and a cardioprotective or hemodynamic drug, wherein the cardioprotective or hemodynamic drug attains a concentration in a subject greater than that which would be attained in normal clinical practice.
2. The composition of item 1, wherein the cardioprotective or hemodynamic drug is selected from the group consisting of Na⁺/K⁺ ATPase inhibitors, calcium channel blockers, β₁-adrenoreceptor agonists, β₁-adrenoreceptor antagonists and thrombolytic agents.
3. The composition of item 1, wherein the cardioprotective or hemodynamic drug decreases heart rate.
4. The composition of item 1, wherein the cardioprotective or hemodynamic drug decreases arrhythmia or vasospasm.
5. The composition of item 1, wherein the cardioprotective or hemodynamic drug decreases fatty acid oxidation.
6. The composition of item 1, wherein the cardioprotective or hemodynamic drug increases contractile force.
7. The composition of item, wherein the cardioprotective or hemodynamic drug increases glucose utilization.
8. The composition of item 1, wherein the cardioprotective or hemodynamic drug increases coronary blood flow.
9. The composition of item 2, wherein the Na⁺/K⁺ ATPase inhibitor is digoxin.
10. The composition of item 2, wherein the calcium channel blocker is diltiazem.
11. The composition of item 2, wherein the β₁-adrenoreceptor agonist is dobutamine.
12. The composition of item 2, wherein the β₁-adrenoreceptor antagonist is metoprolol
13. The composition of item 2, wherein the thrombolytic agent is tissue plasminogen activator (tPA).
14. The composition of item 2, wherein the thrombolytic agent is streptokinase.
15. The composition of item 9, wherein the unit dosage form contains digoxin at a concentration that achieves serum levels greater than 2.5 nM when administered to a subject.
16. The composition of item 9, wherein the unit dosage form contains digoxin at a concentration that achieves serum levels between about 2.5 and 10.0 nM when administered to a subject.
17. The composition of item 10, wherein the unit dosage form contains diltiazem at a concentration that achieves serum levels greater than 0.5 µM when administered to a subject.
18. The composition of item 10, wherein the unit dosage form contains diltiazem at a concentration that achieves serum levels between about 0.5 and 5.0 µM when administered to a subject.
19. The composition of item 11, wherein the unit dosage form contains dobutamine at a concentration that achieves serum levels greater than 0.6 µM when administered to a subject.
20. The composition of item 11, wherein the unit dosage form contains dobutamine at a concentration that achieves serum levels between about 0.6 and 5.0 µM when administered to a subject.
21. The composition of item 12, wherein the unit dosage form contains metoprolol at a concentration that achieves serum levels greater than 0.4 µM when administered to a subject.
22. The composition of item 12, wherein the unit dosage form contains metoprolol at a concentration that achieves serum levels between about 0.4 and 5.0 µM when administered to a subject.
23. A kit comprising the composition of item 1 and instructions for administering the unit dosage form to a subject to produce a cardioprotective or hemodynamic effects.
24. The kit of item 23, wherein the dichloroacetate (DCA) and cardioprotective or hemodynamic drug are individually packaged.
25. The kit of item 23, wherein the dichloroacetate (DCA) and cardioprotective or hemodynamic drug are premixed.
26. The kit of item 23, wherein the cardioprotective or hemodynamic drug is selected from a group consisting of Na⁺/K⁺ ATPase inhibitors, calcium channel blockers, β₁-adrenoreceptor agonists, a β₁-adrenoreceptor antagonists and thrombolytic agents.
27. The kit of item 26, wherein the Na⁺/K⁺ ATPase inhibitor comprises digoxin.
28. The kit of item 26, wherein the calcium channel blocker is diltiazem.
29. The kit of item 26, wherein the β₁-adrenoreceptor agonist is dobutamine.
30. The kit of item 26, wherein the β₁-adrenoreceptor antagonist is metoprolol.
31. The kit of item 26, wherein the thrombolytic agent is tissue plasminogen activator (tPA).
32. The kit of item 26, wherein the thrombolytic agent is streptokinase.
33. A method for treating a subject in need of cardioprotection or of a hemodynamic drug comprising administering DCA and a cardioprotective or hemodynamic drug to the subject, wherein the cardioprotective or hemodynamic drug attains a concentration in the subject greater than that which would be attained in normal clinical practice.
34. The method of item 33, wherein the cardioprotective or hemodynamic drug is selected from a group consisting of Na⁺/K⁺ ATPase inhibitors, calcium channel blockers, β₁-adrenoreceptor agonists, β1- adrenoreceptor antagonists and thrombolytic agents.
35. The method of item 34, wherein the Na⁺/K⁺ ATPase inhibitor is digoxin.
36. The method of item 34, wherein the calcium channel blocker is diltiazem.
37. The method of item 34; wherein the β₁-adrenoreceptor agonist is dobutamine.
38. The method of item 34, wherein the β1-adrenoreceptor antagonist is metoprolol.
39. The method of item 34, wherein the thrombolytic agent is tissue plasminogen activator (tPA).
40. The method of item 34, wherein the thrombolytic agent is streptokinase.
41. The method of item 35, wherein digoxin is at a serum concentration greater than 2.5 nM.
42. The method of 35, wherein digoxin is at a serum concentration between about 2.5 and 10.0 nM.
43. The method of item 36, wherein diltiazem is at a serum concentration greater than 0.5 µM.
44. The method of item 36, wherein diltiazem is at a serum concentration between about 0.5 and 5.0 µM.
45. The method of item 37, wherein dobutamine is at a serum concentration greater than 0.6 µM.
46. The method of item 37, wherein dobutamine is at a serum concentration between about 0.6 and 5.0 µM.
47. The method of item 38, wherein metoprolol is at a serum concentration greater than 0.4 µM.
48. The method of item 38, wherein metoprolol is at a serum concentration between about 0.4 and 5.0 µM.
49. The method of item 33, wherein the cardioprotective or hemodynamic drug decreases heart rate.
50. The method of item 33, wherein the cardioprotective or hemodynamic drug decreases arrhythmia or vasospasm.
51. The method of item 33, wherein the cardioprotective or hemodynamic drug decreases fatty acid oxidation.
52. The method of item 33, wherein the cardioprotective or hemodynamic drug increases contractile force.
53. The method of item 33, wherein the cardioprotective or hemodynamic drug increases glucose utilization.
54. The method of item 33, wherein the cardioprotective or hemodynamic drug increases coronary blood flow.
55. The method of item 33, wherein the subject has or is at risk of hypertension.
56. The method of item 33, wherein the subject has or is at risk of coronary ischemia.
57. The method of item 33, wherein the subject has or is at risk of angina pectoris.
58. The method of item 33, wherein the subject has or is at risk of arrhythmia.
59. The method of item 33, wherein the subject has or is at risk of coronary thrombosis.
60. The method of item 33, wherein the subject has or is at risk of myocardial infarction.
61. The method of item 33, wherein the subject has or is at risk of enlarged heart.
62. The method of item 33, wherein the subject is undergoing a surgical procedure.
63. The method of item 62, wherein the surgical procedure is heart surgery.
64. The method of item 33, wherein the subject is recovering from a surgical procedure.
65. The method of item 64, wherein the surgical procedure is heart surgery.
66. A method of increasing efficacy of a cardioprotective or hemodynamic drug without substantially increasing a side effect caused by the drugs, comprising administering DCA prior to, simultaneously with or following administering the cardioprotective or hemodynamic drug to a subject, wherein the cardioprotective or hemodynamic drug attains a concentration in the subject greater than that which would be attained in normal clinical practice.
67. The method of item 66, wherein the cardioprotective or hemodynamic drug is selected from a group consisting of Na⁺/K⁺ ATPase inhibitors, calcium channel blockers, β₁-adrenoreceptor agonists, β₁-adrenoreceptor antagonists and thrombolytic agents.
68. The method of item 67, wherein the Na⁺/K⁺ ATPase inhibitor is digoxin.
69. The method of item 67, wherein the calcium channel blocker is diltiazem.
70. The method of item 67, wherein the β₁-adrenoreceptor agonist is dobutamine.
71. The method of item 67, wherein the β₁-adrenoreceptor antagonist is metoprolol.
72. The method of item 67, wherein the thrombolytic agent is tissue plasminogen activator (tPA).
73. The method of item 67, wherein the thrombolytic agent is streptokinase.
74. A method of increasing the ability to use a cardioprotective or hemodynamic drug at higher clinical concentrations without substantially increasing a side effect caused by the drugs, comprising administering DCA to a subject prior to, simultaneously with or following administering the cardioprotective or hemodynamic drug, so that the serum concentration of the cardioprotective or hemodynamic drugs in the subject is greater than that used in the absence of DCA.
75. The method of item 74, wherein the cardioprotective or hemodynamic drug is selected from a group consisting of Na⁺/K⁺ ATPase inhibitors, calcium channel blockers, β₁-adrenoreceptor agonists, β₁-adrenoreceptor antagonists and thrombolytic agents.
76. The method of item 75, wherein the Na⁺/K⁺ ATPase inhibitor is digoxin.
77. The method of item 75, wherein the calcium channel blocker is diltiazem.
78. The method of 75, wherein the β₁-adrenoreceptor agonist is dobutamine.
79. The method of item 75, wherein the β₁-adrenoteceptor antagonist is metoprolol
80. The method of item 75, wherein the thrombolytic agent is tissue plasminogen activator (tPA).
81. The method of item 75, wherein the thrombolytic agent is streptokinase
82. A method of treating a subject in need of cardioprotection or of a hemodynamic drug comprising administering DCA to a subject prior to, simultaneously with or following administering the cardioprotective or hemodynamic drug.
83. The method of item 82, wherein the cardioprotective or hemodynamic drug is selected from a group consisting of Na⁺/K⁺ ATPase inhibitors, calcium channel blockers and β₁-adrenoreceptor antagonists.
84. The method of item 82, wherein the β₁-adrenoreceptor antagonist is metoprolol.
85. The method of item 82, wherein the subject would benefit from an increase in cardiac efficiency.
86. The method of item 83, wherein the Na⁺/K⁺ ATPase inhibitor is digoxin.
87. The method of item 83, wherein the calcium channel blocker is diltiazem.
88. The method of item 83, wherein the subject would benefit from a decrease in cardiac oxygen consumption.
89. A composition comprising a unit dosage form of DCA and an effective amount of a cardioprotective or hemodynamic drug.
90. The composition of item 89, wherein the cardioprotective or hemodynamic drug is selected from a group consisting of Na⁺/K⁺ ATPase inhibitors, calcium channel blockers and β₁-adrenoreceptor antagonists.
91. The composition of item 90, wherein the Na⁺/K⁺ ATPase inhibitor is digoxin.
92. The composition of item 90, wherein the calcium channel blocker is diltiazem.
93. The composition of item 90, wherein the β₁-adrenoreceptor antagonist is metoprolol.
94. A kit comprising the composition of item 89 and instructions for administering the unit dosage form to a subject to produce cardioprotective or hemodynamic effects.
95. The kit of item 94, wherein the DCA and cardioprotective or hemodynamic drug are premixed.
96. The kit of item 94, wherein the DCA and cardioprotective or hemodynamic drug are individually packaged.
97. The composition of item 94, wherein the cardioprotective or hemodynamic drugs are selected from a group consisting of Na⁺/K⁺ ATPase inhibitors, calcium channel blockers and β₁-adrenoreceptor antagonists.
98. The kit of item 97, wherein the Na⁺/K⁺ ATPase inhibitor is digoxin.
99. The kit of item 97, wherein the calcium channel blocker is diltiazem.
100. The kit of item 97, wherein the β₁-adrenoreceptor antagonist is metoprolol.

## Claims

1. Use of DCA for the preparation of a pharmaceutical composition for ameliorating the negative side effects of a cardioprotective or hemodynamic drug administered to a subject, for use in the treatment of an ischemic event, wherein the amelioration of negative side effects results from administering an amount of the pharmaceutical composition sufficient to achieve a serum concentration of at least 1mM DCA in the subject.

2. Use of DCA for the preparation of a pharmaceutical composition for ameliorating the negative side effects of a cardioprotective or hemodynamic drug administered to a subject, for use in the treatment of an ischemic event.

3. The use of claim 1 or 2, wherein the cardioprotective or hemodynamic drug is selected from the group consisting of Na+/K+ ATPase inhibitors, calcium channel blockers, β1-adrenoreceptor agonists, β1-adrenoreceptor antagonists; thrombolytic agents, and cardiac glycosides.

4. The use of claim 1 or 2, wherein the cardioprotective or hemodynamic drug decreases heart rate.

5. The use of claim 1 or 2, wherein the cardioprotective or hemodynamic drug decreases arrhythmia or vasospasm.

6. The use of claim 1 or 2, wherein the cardioprotective or hemodynamic drug decreases fatty acid oxidation.

7. The use of claim 1 or 2, wherein the cardioprotective or hemodynamic drug increases contractile force

8. The use of claim 1 or 2, wherein the cardioprotective or hemodynamic drug increases glucose utilization.

9. The use of claim 1 or 2, wherein the cardioprotective or hemodynamic drug increases coronary blood flow.

10. The use of claim 3, wherein the Na+/K+ ATPase inhibitor is digoxin.

11. The use of claim 3, wherein the calcium channel blocker is diltiazem.

12. The use of claim 3, wherein the β1-adrenoreceptor agonist is dobutamine.

13. The use of claim 3, wherein the β1-adrenoreceptor antagonist is metoprolol.

14. The use of claim 3, wherein the thrombolytic agent is tissue plasminogen activator (tPA).

15. The use of claim 3, wherein the thrombolytic agent is streptokinase.

16. The use of claim 3, wherein the cardiac glycoside is a Na+/K+ ATPase inhibitor.

17. The use of claim 10, wherein digoxin is administered in an amount wherein the digoxin attains a serum concentration of greater than 2.5 nM in the subject.

18. The use of claim 10, wherein digoxin is administered in an amount wherein the digoxin attains a serum concentration of between about 2.5 and 10.0 nM in the subject.

19. The use of claim 11, wherein diltiazem is administered in an amount wherein the diltiazem attains a serum concentration of greater than 0.5 µM in the subject.

20. The use of claim 11, wherein diltiazem is administered in an amount wherein the diltiazem attains a serum concentration of between about 0.5 and 5.0 µM in the subject.

21. The use of claim 12, wherein dobutamine is administered in an amount wherein the dobutamine attains a serum concentration of greater than 0.6 µM in the subject.

22. The use of claim 12, wherein dobutamine is administered in an amount wherein the dobutamine attains a serum concentration of between about 0.6 and 5.0 µM in the subject.

23. The use of claim 13, wherein metoprolol is administered in an amount wherein the metropolol attains a serum concentration of greater than 0.4 µM when administered to a subject.

24. The use of claim 13, wherein metoprolol is administered in an amount wherein the metropolol attains a serum concentration of between about 0.4 and 5.0 µM when administered to a subject.

25. The use of any of claims 1 to 24, wherein DCA is administered to the subj ect prior to administration of the cardioprotective or hemodynamic drug.

26. The use of any of claims 1 to 24, wherein DCA is administered to the subject simultaneous with administration of the cardioprotective or hemodynamic drug.

27. The use of any of claims 1 to 24, wherein DCA is administered to the subject subsequent to administration of the cardioprotective or hemodynamic drug.

28. The use of any of claims 1 to 27, wherein the subject is experiencing an ischemic event.

29. The use of any of claims 1 to 27, wherein the subject is recovering from an ischemic event.

30. The use of claims 28 or 29, wherein the subject has hypertension.

31. The use of claims 28 or 29, wherein the subject has coronary ischemia.

32. The use of claims 28 or 29, wherein the subject has angina pectoris

33. The use of claims 28 or 29, wherein the subject has arrhythmia.

34. The use of claims 28 or 29, wherein the subject has coronary thrombosis.

35. The use of claims 28 or 29, wherein the subject has myocardial infarction.

36. The use of claims 29 or 29, wherein the ischemic even results from a surgical procedure.

37. The use of claim 37, wherein the surgical procedure is heart surgery.

38. The use of any of claims 1 to 37, wherein the amelioration of negative side effects results from administering an amount of the pharmaceutical composition sufficient to achieve a serum concentration of at least 2mM DCA in the subject.

39. The use of any of claims 1 to 38, wherein administration of DCA increases glucose metabolism.
